# EUROPÄISCHE PATENTSCHRIFT

(11) **EP 1 242 603 B1**
(45) Veröffentlichungstag und Bekanntmachung des Hinweises auf die Patenterteilung: **21.04.2004**
(21) Anmeldenummer: 00985088.4
(22) Anmeldetag: 23.11.2000
(51) Int. Cl.: C12N 15/81, C12N 15/80, C12N 15/67, C12N 15/90, C12N 1/19, C12P 21/02, C07K 14/62, C07K 14/39

(54) **INTEGRATIONSVEKTOREN UND VERFAHREN ZUR HERSTELLUNG REKOMBINANTER PROTEINE IN PILZEN**
VECTORS AND METHOD FOR PRODUCING RECOMBINANT PROTEINS IN FUNGI
VECTEURS ET PROCEDE DE PRODUCTION DE PROTEINES RECOMBINANTES DANS DES CHAMPIGNONS

(30) Priorität: 23.11.1999 DE 19956297; 03.12.1999 DE 19958327; 29.12.1999 DE 19963690
(43) Veröffentlichungstag der Anmeldung: 25.09.2002
(73) Patentinhaber: RHEIN BIOTECH GESELLSCHAFT FÜR NEUE BIOTECHNOLOGISCHE PROZESSE UND PRODUKTE MBH, 40595 Düsseldorf (DE)
(72) Erfinder: GELLISSEN, Gerd, 42489 Wülfrath (DE); DIESEL, Andre, 47447 Moers (DE); Klabunde, Jens, 40223 Düsseldorf (DE); SUCKOW, Manfred, 40593 Düsseldorf (DE); HOLLENBERG, Cornelis, P., 40237 Düsseldorf (DE)
(74) Vertreter: Grünecker, August, Dipl.-Ing.
(86) Internationale Anmeldenummer: PCT/EP2000/011687
(87) Internationale Veröffentlichungsnummer: WO 2001/038510

(56) Entgegenhaltungen:
- WO-A-99/57279
- ROSSOLINI G M ET AL: "KLUYVEROMYCES-LACTIS RDNA AS A TARGET FOR MULTIPLE INTEGRATION BY HOMOLOGOUS RECOMBINATION" GENE (AMSTERDAM), Bd. 119, Nr. 1, 1992, Seiten 75-81, XP000986022 ISSN: 0378-1119
- YIP C W ET AL: "RIBOSOMAL RNA GENES OF ENDOMYCES FIBULIGER: ISOLATION, SEQUENCING AND THE USE OF THE 26S RRNA GENE IN INTEGRATIVE TRANSFORMATION OF SACCHAROMYCES CEREVISIAE FOR EFFICIENT EXPRESSION OF THE ALPHA-AMYLASE GENE OF ENDOMYCES FIBULIGER" WORLD JOURNAL OF MICROBIOLOGY AND BIOTECHNOLOGY,GB,RAPID COMMUNICATIONS OF OXFORD, OXFORD, Bd. 13, Nr. 1, 1997, Seiten 103-117, XP000677652 ISSN: 0959-3993
- BLANCAFORT PILAR ET AL: "PolI-driven integrative expression vectors for yeast." JOURNAL OF BIOTECHNOLOGY, Bd. 56, Nr. 1, 1997, Seiten 41-47, XP000923345 ISSN: 0168-1656
- FUJII T ET AL: "APPLICATION OF A RIBOSOMAL DNA INTEGRATION VECTOR IN THE CONSTRUCTION OF A BREWER'S YEAST HAVING ALPHA ACETOLACTATE DECARBOXYLASE ACTIVITY" APPLIED AND ENVIRONMENTAL MICROBIOLOGY, Bd. 56, Nr. 4, 1990, Seiten 997-1003, XP000994944 ISSN: 0099-2240
- GELLISSEN GERD ET AL: "Application of yeasts in gene expression studies: A comparison of Saccharomyces cerevisiae, Hansenula polymorpha and Kluyveromyces lactis: A review." GENE (AMSTERDAM), Bd. 190, Nr. 1, 1997, Seiten 87-97, XP002164545 ISSN: 0378-1119
- LOPES T S ET AL: "HIGH-COPY-NUMBER INTEGRATION INTO THE RIBOSOMAL DNA OF SACCHAROMYCES CEREVISIAE: A NEW VECTOR FOR HIGH-LEVEL EXPRESSION" GENE,NL,ELSEVIER BIOMEDICAL PRESS. AMSTERDAM, Bd. 79, Nr. 2, 1989, Seiten 199-206, XP000034807 ISSN: 0378-1119 in der Anmeldung erwähnt
- LOPES T S ET AL: "MECHANISM OF HIGH-COPY-NUMBER INTEGRATION OF PMIRY-TYPE VECTORS INTO THE RIBOSOMAL DNA OF SACCHAROMYCES-CEREVISIAE" GENE (AMSTERDAM), Bd. 105, Nr. 1, 1991, Seiten 83-90, XP000986021 ISSN: 0378-1119 in der Anmeldung erwähnt
- BERGKAMP R J M ET AL: "MULTIPLE-COPY INTEGRATION OF THE ALPHA-GALACTOSIDASE GENE FROM CYAMOPSIS TETRAGONOLOBA INTO THE RIBOSOMAL DNA OF KLUYVEROMYCES LACTIS" CURRENT GENETICS,US,NEW YORK, NY, Bd. 21, Nr. 4/05, 1. April 1992 (1992-04-01), Seiten 365-370, XP002040755 ISSN: 0172-8083 in der Anmeldung erwähnt
- DALL LE M -T ET AL: "MULTIPLE-COPY INTEGRATION IN THE YEAST YARROWIA LIPOLYTICA" CURRENT GENETICS,US,NEW YORK, NY, Bd. 26, Nr. 1, 1994, Seiten 38-44, XP002040754 ISSN: 0172-8083
- HOLLENBERG CORNELIS P ET AL: "Production of recombinant proteins by methylotrophic yeasts." CURRENT OPINION IN BIOTECHNOLOGY, Bd. 8, Nr. 5, 1997, Seiten 554-560, XP000939179 ISSN: 0958-1669

## Beschreibung

Die vorliegende Erfindung betrifft Integrationsvektoren zur gezielten und stabilen Integration heterologer DNA in das Genom eines Wirtsorganismus, insbesondere eines Pilzes, und ihre Verwendung. Femer betrifft die Erfindung ein Verfahren zur Herstellung eines oder mehrerer rekombinanter Proteine unter Verwendung der erfindungsgemäßen Vektoren.

Pilze und insbesondere Hefen stellen attraktive Systeme für die Produktion rekombinanter Proteine dar. Ein prominenter Vertreter ist die fakultativ methylotrophe Hefe *Hansenula polymorpha*, die für die Produktion von pharmazeutischen Substanzen und industriellen Enzymen genutzt wird. Die Generierung rekombinanter Stämme von *H. polymorpha* erfolgt routinemäßig mit Vektoren, die in das Genom der Hefe integriert werden. Aufgrund der genomischen Lokalisation der heterologen DNA zeichnen sich die rekombinanten Stämme von *H. polymorpha* durch eine hohe mitotische Stabilität aus. Jeder Transformationsansatz resultiert jedoch in unterschiedlichen rekombinanten Stammen, die sich in Integrationsort und Kopienzahl der integrierten heterologen DNA unterscheiden; wobei die Kopienzahl von 1 bis 80 variieren kann. Da der Integrationsort zufällig ist, wird für die Integration eine nicht-homologe Rekombination in nicht näher charakterisierte Genloci angenommen.

Die zielgerichtete Integration von heterologer DNA in das Genom von *H. polymorpha* hat sich dagegen als sehr schwierig erwiesen. In jüngerer Zeit wurden erste Möglichkeiten für eine gezielte homologe Integration in definierte Genorte beschrieben. Diese wenigen Beispiele sind auf einen Disruptions-Integrations-Ansatz beschränkt, bei dem die rekombinante DNA zielgerichtet in den *MOX* (Methanol oxidase)-Locus (Agaphonov *et al*., 1995) oder in Telomer-assoziierte *HARS*-Sequenzen von *H. polymorpha* (Sohn *et al*., 1999) integriert wurde.

Von Vorteil wäre eine zielgerichtete homologe Integration in einen Genlocus, der gute Voraussetzungen für Exprimierbarkeit bietet. Dies gilt in der Regel für solche Bereiche eines Chromosoms, die ein stark exprimiertes Gen enthalten, wie z.B. das oben angeführte *MOX*-Gen. Für eine zielgerichtete Integration von Fremdsequenzen wurde ferner ein hochexprimierter Genombereich, der die Gene für die ribosomalen RNAs enthält (die sogenannte rDNA) in Betracht gezogen.

Dieser Ansatz wurde für zwei Hefearten, die Bierhefe *Saccharomyces cerevisiae* (Lopes *et al*., 1989; Lopes *et al*., 1991) und die Milchhefe *Kluyveromyces lactis* (Bergkamp *et al*., 1992) getestet. Die Integration erfolgte in diesen Fällen durch homologe Rekombination mit Hilfe von rDNA-Fragmenten des entsprechenden Wirtes. Auf diese Weise generierte rekombinante Stämme enthielten multiple Kopien der heterologen DNA integriert in den rDNA-Locus der Transformanden. Als ein wichtiger, unabdingbarer Faktor für die Integration des Vektors in hoher Kopienzahl wurde die Anwesenheit eines Komplementationsgens mit einem defizienten Promotor (d.h. *Leu2-d* oder *Ura3-d*) mit einer hohen selektiven Wirkung genannt. Die Kombination eines defizienten Promotors und eines definierten rDNA-Segments erwies sich als unverzichtbar.

Die Generierung von rekombinanten Stämmen mit unterschiedlichen Fremdgenen in einer erwünschten Kopienzahl war bisher nur durch aufeinanderfolgende Transformationsschritte möglich. Dazu müssen die genutzten Plasmide unterschiedliche Selektionsmarkergene aufweisen. Beispiele dafür sind Stämme für die Koexpression der Gene für das HbsAg-L- und -S-Antigen (Janowicz *et al*., 1991), für die Entwicklung eines Hepatitis B-Impfstoffes oder die Koexpression von zwei Enzymgenen für die Entwicklung eines Biokatalysators (Gellissen *et al*., 1996). Es besteht also ein Bedarf an Vektoren, die die simultane Integration verschiedener heterologer DNA-Sequenzen in das Genom eines Wirtsorganismus erlauben.

Aufgabe der Erfindung ist es daher, weitere Integrationsvektoren bereitzustellen, die es erlauben, die Fremd-DNA in hoher Kopienzahl in das Genom eines Wirtsorganismus stabil zu integrieren und bei Bedarf unterschiedliche Gene für eine simultane Koproduktion verschiedener Proteine zu exprimieren. Eine weitere Aufgabe der Erfindung ist es, stabile Produktionsstämme und ein Verfahren zur Herstellung heterologer Proteine bereitzustellen.

Diese Aufgabe wird durch einen Integrationsvektor gelöst, welcher
a) mindestens eine rDNA-Sequenz aus Hefe,
b) mindestens ein nicht-defizientes Selektionsmarkergen und
c) mindestens eine Expressionskassette
umfaßt, wobei die Expressionskassette ein im Wirtsorganismus funktionsfähiges Promotorelement, einen für ein gewünschtes Protein kodierenden Bereich eines heterologen oder genuinen Genes und ein im Wirtsorganismus funktionsfähiges Terminatorelement umfasst, dadurch gekennzeichnet, dass mindestens eine rDNA-Sequenz aus einem Bereich der rDNA einer methylotrophen Hefe stammt, der ein 400 bp langes Fragment aus dem 3'-Bereich des 25S―rDNA-Gens und ein 600 bp langes Fragment aus dem 5'-Bereich des 18S-rDNA-Gens sowie die dazwischen liegenden Sequenzen einschließlich der 5S-rDNA-Sequenz umfasst.

Überraschenderweise hat sich herausgestellt, daß eine stabile Multi-Copy-Integration eines Integrationsvektors mit mindestens einer rDNA-Sequenz aus Hefe wie vorhergehend beschrieben unter Verzicht auf ein defizientes Selektionsmarkergen erreicht werden kann, und daß verschiedene Integrationsvektoren mit identischen, nicht defizienten Markergenen gleichzeitig integriert werden können. Bei der gleichzeitigen Integration mehrerer Vektoren bleibt die Stöchiometrie mitotisch stabil erhalten. Die Etablierung eines "universellen" Hefevektors, basierend auf den neu identifizierten Elementen, erlaubt eine breite Anwendung der Erfindung.

Unter einer "rDNA-Sequenz aus Hefe" wird im Sinne der Erfindung eine Sequenz verstanden, die aus einem Abschnitt des Genoms einer Hefe, der die Gene für die ribosomalen RNAs enthält, hergeleitet ist. Zur Ausführung der Erfindung in Betracht kommende rDNA-Sequenzen stammen unter anderem aus Hefen der Gattungen *Hansenula, Pichia, Saccharomyces, Kluyveromyces, Candida, Schwanniomyces, Yarrowia, Arxula* oder *Trichosporon,* und insbesondere aus den methylotrophen Hefen *Hansenula polymorpha* und *Pichia pastoris*.

Ein "nicht-defizientes Selektionsmarkergen", wie hier verwendet, bezeichnet ein Gen, dessen Proteinprodukt dem Wirtsorganismus bestimmte selektierbare Eigenschaften verleiht, die er vor der Transformation und Integration des erfindungsgemäßen Vektors bzw. der erfindungsgemäßen Vektoren nicht besaß (beispielsweise Resistenz gegen ein Antibiotikum, Komplementation einer Auxotrophie), wobei die Aktivität des von einer einzigen Kopie des Selektionsmarkergens kodierten Produkts für ein normales Wachstum des Wirtsorganismus ausreicht. Beispiele für nicht-defiziente Selektionsmarkergene, die für die Ausführung der Erfindung in Betracht kommen, sind unter anderem die *URA3-, HIS3-*, *ADE2-*, *LYS2-, ARO7-* und *TRP1*-Gene von *S. cerevisiae* und das *LEU2*-Gen von *H. polymorpha*. Alternativ kann ein dominantes Selektionsmarkergen wie z.B. das bakterielle Kanamycinresistenz-Gen eingesetzt werden.

"Expressionskassette", wie hier verwendet, bezeichnet ein DNA-Fragment, das in mindestens eine mRNA transkribiert wird, die nachfolgend in ein Genprodukt translatiert wird. Erfindungsgemäß Umfaßt die Expressionskassette ein im Wirtsorganismus funktionsfähiges Promotorelement, einen für ein gewünschtes Protein kodierenden Bereich eines heterologen Genes und ein im Wirtsorganismus funktionsfähiges Terminatorelement. Die Expressionskassette kann ferner eine Sequenz für ein Signalpeptid und/oder eine Polylinker-Sequenz enthalten. Die vorgenannten Bestandteile einer Expressionskassette können aus einem Gen oder aus verschiedenen Genen stammen.

Ein "im Wirtsorganismus funktionsfähiges Promotorelement", wie hier verwendet, bezeichnet ein DNA-Fragment, durch das der Initiationspunkt und die Initiationshäufigkeit der Transkription (RNA-Synthese) eines unter der Kontrolle des Promotorelements stehenden Gens im Wirtsorganismus festgelegt werden. Zur Ausführung der Erfindung in Betracht kommende Promotorelemente schließen unter anderem die *FMD-*, *MOX-*, *TPS1-*, *PMA1-* und DAS-Promotoren aus *Hansenula polymorpha*, die *MOX-, AOX1-* und *GAP1*-Promotoren aus *Pichia pastoris* und die *ADH1-, PDC1-, GAP1-* und *CUP1*-Promotoren aus *S. cerevisiae* ein.

Ein "heterologes Gen", wie hier verwendet, bezeichnet ein Gen, das von einem anderen Organismus stammt als dem Wirtsorganismus, in dem es exprimiert werden soll. Beispiele für exprimierbare heterologe Gene sind unter anderem die Gene für Insulin, Phytase, HGH, Hirudin, Lactoferrin oder G-CSF. Als "genuines Gen" im Sinne der Erfindung wird ein Gen bezeichnet, das aus einem Organismus derselben Art wie der Wirtsorganismus, in dem das Gen exprimiert werden soll, stammt.

Ein "im Wirtsorganismus funktionsfähiges Terminatorelement", wie hier verwendet, bezeichnet ein DNA-Fragment, das Signalstrukturen für die RNA-Polymerase enthält, die zur Termination der Transkription führen. Beispiele für verwendbare Terminatorelemente sind der *MOX-* oder der *PHO1*-Terminator von *H. polymorpha*.

Als Wirtsorganismen zur Ausführung der vorliegenden Erfindung kommen insbesondere Pilze in Betracht, beispielsweise filamentöse Pilze wie etwa *Aspergillus, Neurospora, Mucor, Trichoderma, Acremonium, Sordaria* und *Penicillium* oder Hefen wie *Saccharomyces, Hansenula, Pichia, Kluyveromyces, Schwanniomyces, Yarrowia, Arxula, Trichosporon* und *Candida.* Vorzugsweise ist der Wirtsorganismus, in dem die Expression heterologer Gene zur Herstellung von einem oder mehreren Proteinen mit Hilfe der erfindungsgemäßen Integrationsvektoren stattfindet, eine Hefe, insbesondere eine methylotrophe Hefe. In der bevorzugten Ausführungsform der Erfindung werden die erfindungsgemäßen Integrationsvektoren für die Herstellung von Proteinen in einer Hefe der Gattung *Hansenula,* vorzugsweise *Hansenula polymorpha*, eingesetzt.

Die in dem erfindungsgemäßen Vektor enthaltene rDNA-Sequenz ist aus einem Abschnitt des Genoms einer Hefe hergeleitet, der die Gene für die ribosomalen RNAs enthält. Eine solche rDNA-Sequenz kann beispielsweise einen etwa 1 Kb langen Abschnitt aus dem 3'-Bereich des 25S-rDNA-Gens und einen etwa 1 Kb langen Abschnitt aus dem 5'-Bereich des 18S-rDNA-Gens sowie die dazwischen liegenden Sequenzen einschließlich der 5S-rDNA-Sequenz umfassen. Wie hier verwendet, beziehen sich die Angaben "Abschnitt aus dem 3'-Bereich des 25S-rDNA-Gens" und "Abschnitt aus dem 5'-Bereich des 18S-rDNA-Gens" auf kodierende Bereiche des 25S- bzw. 18S-rDNA-Gens. Ein rDNA-Fragment, das mittels PCR mit genomischer DNA einer Hefe, z.B. einer methylotrophen Hefe wie *Hansenula polymorpha*, als Matrize und den Primem A (5'-ACG GGA TCC GGG TTT AGA CCG TCG TGA GAC AGG-3'; SEQ ID NO. 2) und B (5'-CCT GGA TCC TAC CAT CGA AAG TTG ATA GGG-3'; SEQ ID NO. 3) amplifiziert und durch Verdau mit dem Restriktionsenzym *Bam*HI erhältlich ist, eignet sich ebenfalls als rDNA-Sequenz im Sinne dieser Erfindung.

Als besonders vorteilhaft haben sich erfindungsgemäße Integrationsvektoren erwiesen, bei denen mindestens eine rDNA-Sequenz aus einem Bereich der rDNA einer methylotrophen Hefe stammt, der ein 400 bp langes Fragment aus dem 3'-Bereich des 25S-rDNA-Gens und ein 600 bp langes Fragment aus dem 5'-Bereich des 18S-rDNA-Gens sowie die dazwischen liegenden Sequenzen einschließlich der 5S-rDNA-Sequenz umfaßt. Wie hier verwendet, beziehen sich die Angaben "Fragment aus dem 3'-Bereich des 25S-rDNA-Gens" und "Fragment aus dem 5'-Bereich des 18S-rDNA-Gens" auf kodierende Bereiche des 25S- bzw. 18S-rDNA-Gens.Geht man von einer ähnlichen rDNA-Struktur wie in *Saccharomyces cerevisiae* aus, so enthält dieser ca. 3 Kb lange Bereich eine TOP-Sequenz, eine oder mehrere HOT1-Sequenzen sowie NTS1- und NTS2-Sequenzen. Erfindungsgemäße Vektoren, die eine solche rDNA-Sequenz enthalten, werden in hoher Kopienzahl und stabil integriert. Bevorzugt stammen diese rDNA-Sequenzen aus *Hansenula polymorpha*. Altemativ können rDNA-Sequenzen aus anderen methylotrophen Hefen wie z. B. *Pichia pastoris, Pichia methanolica* oder *Candida boidinii* verwendet werden.

In einer bevorzugten Ausführungsform der Erfindung umfassen die erfindungsgemäßen Integrationsvektoren mindestens eine rDNA-Sequenz, die aus folgenden Sequenzen ausgewählt wird:
a) einer Sequenz nach SEQ ID NO 1;
b) einem Fragment der Sequenz nach a), das für die stabile Integration von Sequenzen des Vektors in hoher Kopienzahl in das Genom eines Wirtsorganismus, insbesondere einer Hefe geeignet ist;
c) einer Sequenz, die mit einer Sequenz nach a) oder b) zu mindestens 70% identisch ist;
d) einer Sequenz, die in dem kodierenden Bereich des 5S-Gens (Positionen 956-1074 in SEQ ID NO 1) und/oder in dem in SEQ ID NO 1 enthaltenen Abschnitt des kodierenden Bereichs des 25S-Gens (Positionen 1977-2420 in SEQ ID NO 1) mit einer Sequenz nach a) oder b) zu mindestens 70% identisch ist;
e) einer Sequenz, die mit dem Gegenstrang einer Sequenz nach a) oder b) hybridisiert;
f) einem durch Substitution, Addition, Inversion und/oder Deletion einer oder mehreren Basen erhaltenen Derivat einer Sequenz nach einer der Gruppen a) bis d), das für die stabile Integration von Sequenzen des Vektors in hoher Kopienzahl in das Genom eines Wirtsorganismus, insbesondere einer Hefe geeignet ist, und
g) einer zu einer Sequenz nach einer der Gruppen a) bis f) komplementären Sequenz.

Die 2,4 Kb lange, als *Bam*HI/*Eco*RI-Fragment isolierbare rDNA-Sequenz nach SEQ ID NO 1 umfaßt 400 bp des 3'-Bereiches des 25S-rDNA-Gens sowie TOP-, HOT1-, NTS1-und NTS2-Sequenzen, die das 5S-rDNA-Gen flankieren. Zur Ausführung der Erfindung können auch Fragmente dieser Sequenz eingesetzt werden, die für die stabile Integration von Sequenzen des Vektors in hoher Kopienzahl in das Genom eines Wirtsorganismus, insbesondere einer Hefe geeignet sind, beispielsweise ein Fragment, das keine Sequenzen des 25S-rDNA-Gens enthält, oder ein Fragment, das die zwischen dem 3'-Ende des 25S-Gens und dem 5'-Ende des 5S-Gens gelegenen TOP-, HOT1-, NTS- und 5S-Sequenzen umfaßt. Für die Ausführung der Erfindung kommen ebenfalls Fragmente in Betracht, in denen eine oder mehrere der HOT- und/oder NTS-Sequenzen fehlen, sowie Fragmente, in denen die TOP- und/oder die 5S-rDNA-Sequenz fehlt. Da jedoch angenommen wird, daß die TOP- und HOT-Sequenzen wichtige Bestandteile der in den erfindungsgemäßen Integrationsvektoren enthaltenen rDNA-Sequenz sind, werden Fragmente der SEQ ID NO 1 bevorzugt, die mindestens eine TOP-Sequenz und mindestens eine HOT-Sequenz enthalten.

Der Ausdruck "stabile Integration", wie hier verwendet, bezeichnet eine Integration, bei der Restriktionsmuster und Kopienzahl nach mindestens 100 Generationen Dauerkultur in nicht-selektivem Medium im wesentlichen unverändert bleiben.

Vorzugsweise ist die Sequenz nach Gruppe d) mit einer Sequenz nach der Gruppe a) oder b) in dem kodierenden Bereich des 5S-Gens (Positionen 956-1074 in SEQ ID NO 1) und/oder in dem in SEQ ID NO 1 enthaltenen Abschnitt des kodierenden Bereichs des 25S-Gens (Positionen 1977-2420 in SEQ ID NO 1) zu mindestens 80%, besonders bevorzugt zu mindestens 90% identisch.

Im Sinne der Erfindung beziehen sich die Ausdrücke "mindestens 70% identisch", "mindestens 80% identisch" und "mindestens 90% identisch" auf Identität auf DNA-Ebene, die gemäß bekannter Verfahren, z.B. der computergestützten Sequenzvergleiche (Basic local alignment search tool, S.F. Altschul *et al*., J. Mol. Biol. 215 (1990), 403-410) bestimmt werden kann.

Der dem Fachmann bekannte Ausdruck "Identität" bezeichnet den Grad der Verwandtschaft zwischen zwei oder mehr DNA-Molekülen, der durch die Übereinstimmung zwischen den Sequenzen bestimmt wird. Der Prozentsatz der "Identität" ergibt sich aus dem Prozentsatz identischer Bereiche in zwei oder mehr Sequenzen unter Berücksichtigung von Lücken oder anderen Sequenzbesonderheiten.

Die Identität miteinander verwandter DNA-Moleküle kann mit Hilfe bekannter Verfahren bestimmt werden. In der Regel werden spezielle Computerprogramme mit den besonderen Anforderungen Rechnung tragenden Algorithmen eingesetzt. Bevorzugte Verfahren zur Bestimmung der Identität erzeugen zunächst die größte Übereinstimmung zwischen den untersuchten Sequenzen. Computerprogramme zur Bestimmung der Identität zwischen zwei Sequenzen umfassen, sind jedoch nicht eingeschränkt auf, das GCG-Programmpaket, einschließlich GAP (Devereux, J., *et al*., Nucleic Acids Research 12 (12): 387 (1984); Genetics Computer Group University of Wisconsin, Madison, (WI)); BLASTP, BLASTN und FASTA (Altschul, S. *et al*., J. Molec Biol 215:403/410 (1990)). Das BLAST X Programm kann vom National Centre for Biotechnology Information (NCBI) und aus weiteren Quellen bezogen werden (BLAST Handbuch, Altschul S., *et al*., NCB NLM NIH Bethesda MD 20894; Altschul, S., *et al*., J. Mol. 215:403/410 (1990)). Auch der bekannte Smith Waterman-Algorithmus kann zur Bestimmung von Identität verwendet werden.

Bevorzugte Parameter für den Sequenz-Vergleich umfassen die nachstehenden:
- Algorithmus:: Needleman und Wunsch, J. Mol. Biol 48:443-453 (1970)
- Vergleichsmatrix:: Übereinstimmung (matches) = + 10,
Nichtübereinstimmung (mismatch) = 0
- Lücken-Wert (Gap Penalty):: 15
- Lückenlängen-Wert: (Gap Length Penalty):: 1

Das GAP-Programm ist auch zur Verwendung mit den vorstehenden Parametern geeignet. Die vorstehenden Parameter sind die Standardparameter (default parameters) für Nukleinsäuresequenz-Vergleiche.

Weitere beispielhafte Algorithmen, Lücken-Öffnungs-Werte (gap opening penalties), Lückenausdehnungs-Werte (gap extension penalties), Vergleichsmatrizen einschließlich der im Programm-Handbuch, Wisconsin-Paket, Version 9, September 1997, genannten können verwendet werden. Die Auswahl wird von dem durchzuführenden Vergleich abhängen und weiterhin davon, ob der Vergleich zwischen Sequenzpaaren, wobei GAP oder Best Fit bevorzugt sind, oder zwischen einer Sequenz und einer umfangreichen Sequenz-Datenbank, wobei FASTA oder BLAST bevorzugt sind, durchgeführt wird.

Das Merkmal "Sequenz, die mit dem Gegenstrang einer Sequenz nach a) oder d) hybridisiert" weist auf eine Sequenz hin, die unter stringenten Bedingungen mit dem Gegenstrang einer Sequenz mit den unter a) oder b) angegebenen Merkmalen hybridisiert. Beispielsweise können die Hybridisierungen bei 68°C in 2 x SSC oder nach dem Protokoll des Dioxygenin-Labelling-Kits der Firma Boehringer (Mannheim) durchgeführt werden.

In einer Ausführungsform der Erfindung umfassen die erfindungsgemäßen Integrationsvektoren ein zusätzliches Selektionsmarkergen für *E*. *coli*, beispielsweise ein Ampicillin-Resistenzgen, ein Tetracyclin-Resistenzgen oder ein Kanamycin-Resistenzgen. Als Selektionsmarkergen für *E. coli* kommt ebenfalls das *URA3*-Gen aus *S. cerevisiae* in Betracht, das eine Selektion von Transformanden des Uracil-auxotrophen *E. coli*-Stamms MC1066 erlaubt.

Vorzugsweise weisen die erfindungsgemäßen Vektoren eine Länge von 7 bis 12 Kb auf. Solche Vektoren bieten eine gute Handhabung in *E. coli* und eine problemlose Transformation von Hefe.

Die erfindungsgemäßen Integrationsvektoren können Sequenzen enthalten, die ein oder mehrere Polypeptide kodieren, die eine verbesserte Sezemierung eines kointegrierten rekombinanten sekretorischen Proteins aus einem Wirtsorganismus ermöglichen. Solche Sequenzen sind beispielsweise Sequenzen, die ein Polypeptid mit der biologischen Aktivität einer Ca²⁺/Calmodulin-abhängigen Proteinkinase und/oder ein Polypeptid mit der biologischen Aktivität eines Translationsinitiationsfaktors 4E (eIF4E) kodieren, und insbesondere Sequenzen, die den kodierenden Bereich des *CMK2*-Gens und/oder den kodierenden Bereich des *CDC33*-Gens aus *Saccharomyces cerevisiae* umfassen und das vollständige CMK2- und/oder eIF4E-Protein kodieren. Sequenzen, die Polypeptide kodieren, die zur Überwindung von bei der Sezemierung eines Proteins aus einer eukaryontischen Zelle auftretenden Engpässen beitragen, können mit dem in DE 199 20 712 A1 beschriebenen Verfahren identifiziert werden.

Ein Wirtsorganismus, der mindestens einen erfindungsgemäßen Integrationsvektor stabil im Genom integriert umfaßt, wird von der Erfindung ebenfalls bereitgestellt. Der Wirtsorganismus kann ein filamentöser Pilz sein, beispielsweise ein Pilz der Gattungen *Aspergillus, Neurospora, Mucor, Trichoderma, Acremonium, Sordaria* oder *Penicillium*. Vorzugsweise ist der Wirtsorganismus eine Hefe, beispielsweise eine Hefe der Gattungen *Hansenula, Pichia, Saccharomyces, Kluyveromyces, Candida, Schwanniomyces, Yarrowia, Arxula* oder *Trichosporon*. In einer Ausführungsform der Erfindung ist der Wirtsorganismus eine methylotrophe Hefe, vorzugsweise eine Hefe der Gattung *Hansenula;* die methylotrophe Hefe *Hansenula polymorpha* ist als Wirtsorganismus besonders bevorzugt.

Die Erfindung stellt ferner einen Wirtsorganismus bereit, der durch stabile Integration von einem oder mehreren erfindungsgemäßen Integrationsvektoren in das Genom eines Pilzes erhältlich ist. Der Wirtsorganismus kann ein filamentöser Pilz, vorzugsweise ein Pilz der Gattung *Aspergillus, Neurospora, Mucor, Trichoderma, Acremonium, Sordaria* oder *Penicillium,* oder eine Hefe sein. Vorzugsweise gehört die Hefe der Gattung *Hansenula,* *Pichia, Saccharomyces, Kluyveromyces, Candida, Schwanniomyces, Yarrowia, Arxula* oder *Trichosporon* an. In einer besonders bevorzugten Ausführungsform der Erfindung ist die Hefe eine methylotrophe Hefe; besonders bevorzugt ist die methylotrophe Hefe *Hansenula polymorpha*. In einer besonderen Ausführungsform der Erfindung ist der Wirtsorganismus durch stabile Integration mehrerer Vektoren, die voneinander unterschiedliche Expressionskassetten umfassen, erhältlich.

Die Erfindung stellt weiterhin ein Verfahren zur Herstellung von einem oder mehreren Proteinen in einem eukaryontischen Mikroorganismus bereit. Erfindungsgemäß wird dafür ein erfindungsgemäßer Wirtsorganismus in an sich bekannter Weise kultiviert und das erzeugte Protein gewonnen.

Die Verwendung der erfindungsgemäßen Integrationsvektoren und Wirtsorganismen zur Herstellung von einem oder mehreren Proteinen wird von der Erfindung ebenfalls umfaßt.

Die folgenden Figuren und Beispielen erläutern die Erfindung.

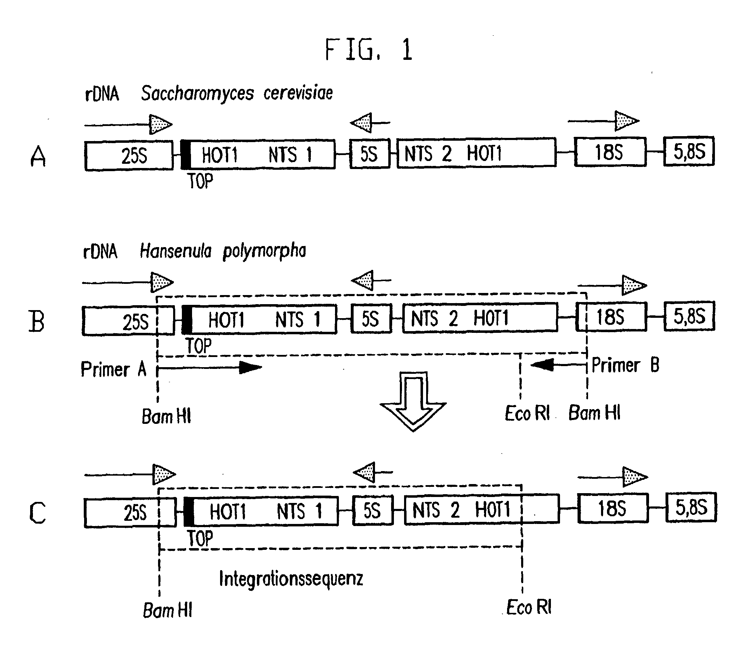

Figur 1 zeigt die Organisation einer rDNA-Einheit von *S. cerevisiae* (A) und *H. polymorpha* (B) sowie die davon abgeleitete Position der klonierten rDNA-Sequenz (SEQ ID NO 1) in einer rDNA-Einheit von *H. polymorpha*. Die grauen Pfeile kennzeichnen die Transkriptionsrichtung der rRNA-Gene. Die gestrichelten Kästen kennzeichnen das primäre Amplifikat und die in den Integrationsvektoren genutzte Integrationssequenz, wobei die Darstellung nicht maßstabgerecht ist. NTS = Non-transcribed spacer; 5S = 5S-rDNA; HOT = Hot spot of recombination; TOP = Topoisomerase-Bindestelle. (A) Die rDNA-Einheit von *S. cerevisiae* nach Lopes *et al*. (1996). (B) Position des primären Amplifikates. Die zu *S. cerevisiae* homologen Primer binden an die hoch konservierte 25S-rDNA (Primer A) und an das 5'-Ende der 185-rDNA (Primer B) innerhalb der rDNA-Einheit von *H. polymorpha*. Die schwarzen Pfeile kennzeichnen das primäre PCR-Amplifikat. (C) Position der 2,4 Kb langen Integrationssequenz (SEQ ID NO 1) in der rDNA-Einheit von *H. polymorpha*. Diese Sequenz beinhaltet das 5S-Gen, die NTS1-Sequenz und Teilbereiche der NTS2-Sequenz und 25S-Sequenz.
Figur 2 stellt die 2,4 Kb lange rDNA-Sequenz eines Integrationselementes (SEQ ID NO 1) eines erfindungsgemäßen rDNA-Integrationsvektors dar. Der Bereich der 5S-rDNA ist grau schattiert.
Figur 3 stellt den rDNA-Integrationsvektor pM2 dar.
Figur 4 stellt den rDNA-Integrationsvektor pM2Δ dar.
Figur 5 stellt pM2ZT (*lacZ*, *TPS1*-Promotor), einen rDNA-Integrationsvektor zur Expression von *lacZ* in *H. polymorpha* dar.
Figur 6 stellt pM2GT (GFP3,*TPS1*-Promotor), einen rDNA-Integrationsvektor zur Expression von GFP in *H. polymorpha* dar.
Figur 7 zeigt das Ergebnis einer *Southern Blot*-Analyse zum Nachweis der Integration von pB14 in die rDNA von *H. polymorpha*.
Figur 8 zeigt ein Modell für die Integration eines rDNA-Integrationsvektors (pB14) in die DNA. Die Figur zeigt die Zuordnung der Fragmente aus der Southem-Blot Analyse in Figur 7. Aus der Restriktionstelle für *Bgl*II und den bekannten Längen der Fusionsfragmente aus heterologer DNA und rDNA ergibt sich die Zuordnung der Fragmentlängen aus der *Southern Blot*-Analyse.
Figur 9 zeigt das Ergebnis einer *Southern Blot*-Analyse für eine Auswahl rekombinanter Stämme nach Transformation von RB11 mit drei verschiedenen Vektoren.

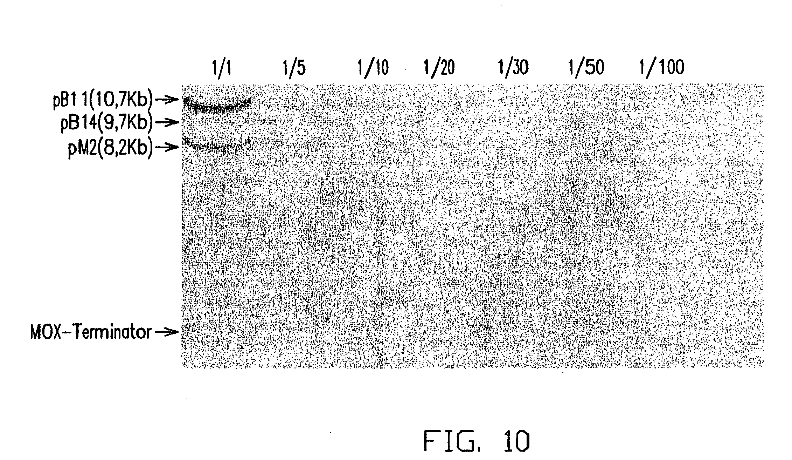

Figur 10 zeigt das Ergebnis einer *Southern Blot*-Analyse zur Bestimmung der Kopienzahl der kointegrierten Vektoren in dem rekombinanten Stamm RB3T-19.
Figur 11 zeigt das Ergebnis einer *Southern Blot*-Analyse zum Nachweis der mitotischen Stabilität des rekombinanten Stammes RB3T-19. Die Ziffern kennzeichnen den Tag der Probenentnahme.
Figur 12 zeigt eine *Southern Blot*-Analyse von vier rekombinanten Stämmen, die durch gleichzeitige Transformation mit fünf Vektoren generiert wurden. Drei dieser Stämme enthalten Sequenzen von mindestens einem rDNA-Integrationsvektor.
Figur 13 zeigt die durch den *TPS1*-Promotor kontrollierte Expression von *lacZ* in rekombinanten Stämmen von *H. polymorpha*. Die Kolonien sind von links oben nach rechts unten durchgehend numeriert.
Figur 14 zeigt die Identifizierung von rekombinanten Insulin-sezemierenden Stämmen von *H. polymorpha* in einem immunologischen Filtertest. Die Ziffern auf den Filtern entsprechen den Nummern der rekombinanten Stämme. Filter A zeigt die Stämme RB4T 1-66.

Die Positivkontrolle befindet sich an Position 67. Filter B zeigt die Stämme RB4T 67-132. Die Positivkontrolle befindet sich an Position 133.

### Beispiele

### Material und Methoden

### 1. Abkürzungen

- amp: Ampicillin
- amp^{R}: Ampicillin-Resistenz
- ARS: Autonomously Replicating Sequence
- BSA: Rinderserumalbumin (Bovine Serum Albumin)
- Bp: Basenpaare
- DNA: Desoxyribonukleinsäure
- DTT: Dithiothreitol
- EDTA: Ethylendiamintetraessigsäure
- ELISA: Enzyme Linked Immuno Assay
- *FMD*: Gen für die Formiat-Dehydrogenase
- HARS: *H. polymorpha* Autonomously Replicating Sequences
- HOT: Hot spot of recombination
- Kb: Kilobasenpaare
- LB: Luria-Broth-Medium
- *MOX*: Gen für die Methanol-Oxidase aus *Hansenula polymorpha*
- NTS: Non-transcribed spacer
- ori: Replikationsursprung eines Plasmides (origin of replication)
- ODₓ: Optische Dichte bei ₓ nm Wellenlänge
- PCR: Polymerase Chain Reaction
- pH: potentia Hydrogenii
- rDNA: ribosomale DNA
- rRNA: ribosomale RNA
- rpm: Umdrehungen pro Minute (*rounds per minute*)
- SDS: Natriumdodecylsulfat (Sodium Dodecyl Sulphate)
- TOP: Topoisomerase-Bindestelle
- *TPS1*: Gen für Trehalose-6-Phosphat-Synthase
- Tris: Tris-(hydroxymethyl)-Methylamin
- ÜK: Übemachtkultur
- X-Gal: 5-Brom-4-chlor-3-indolyl-β-D-Galactopyranosid
- YNB: Yeast Nitrogen Base (Selektionsmedium ohne Uracil).
- YPD: Vollmedium

### 2. Chemikalien und Enzyme

Chemikalien wurden, soweit nicht besonders erwähnt, von folgenden Herstellern bezogen: Acros Organics (New Jersey); Amersham International plc. (Buckinghamshire, England); Baker (Deventer, Niederlande); Biorad (Richmond, USA); Biozym (Hess. Oldendorf); Caesar & Loretz GmbH (Hilden); Difco-Laboratories (Detroit, USA); Fluka AG (Basel, Schweiz); Gibco (Paisly, Schottland); Janssen (Beerse, Belgien); Merck (Darmstadt); Oxoid Ltd. (Basingstone, England); Pharmacia (Freiburg); Riedel de Haen (Seelze); Roth (Karisruhe); Serva (Heidelberg); Sigma (St.Louis, USA); Restriktionsenzyme wurden von den Herstellern Boehringer (Mannheim), Gibco BRL (Paisly, Schottland) und New England Biolabs (Schwalmbach) bezogen. Es wurde in den jeweilig mitgelieferten Puffern gearbeitet. Das Enzym Zymolyase wurde von der Seikaguko Corp. bezogen. DNA-Präparationen wurden mit Hilfe verschiedener DNA-Isolationskits der Firma Quiagen (Hilden) nach den Angaben des Herstellers durchgeführt.

### 3. Stämme

### 3.1 E. coli

- DH5αF': F'endA1*hsd*R17rₖmₖ+*supE44thi*-*1recA1gyra relA(lacZYA-argF*) U169(φ80Δ(lacZ)M15) (Gibco BRL, Gaithersburg MD, USA)
- MC 1066:: Δ (lacI POZYA) Δ74, galU, galK, strA⁻, usdR⁻, trpC 9830, leu B6, pyr F74::Tn5 (Km^{r}) (Casadaban *et al*., 1983).

### 3.2 H. polymorpha

RB11 *odc1* Orotidin-5-phosphat-Decarboxylase-defizienter (Uracil-auxotropher) *H. polymorpha*-Stamm (Weydemann *et al*., 1995).

### 4. Medien

### 4.1 Medien zur Kultivierung von H. polymorpha

Vollmedium: 2% Glucose oder Glycerin, 1% Hefeextrakt, 2% Bacto-Peptone. Selektionsmedium: 0,17% Yeast Nitrogen Base, 0,5% Ammoniumsulfat, 2% Glucose oder Glycerol, 38,4 mg/l Arginin, 57,6 mg/l Isoleucin, 48 mg/l Phenylalanin, 57,6 mg/l Valin, 6 mg/ml Threonin, 50 mg/l Inositol, 40 mg/l Tryptophan, 15 mg/l Tyrosin, 60 mg/l Leucin, 4 mg/l Histidin. Uracil fehlt im Selektionsmedium.

### 4.2 Medien zur Kultivierung von E. coli

Luria Broth-Medium (LB): 10 g/l Casein-Hydrolysat (Pepton No.140, Gibco), 5 g/l Hefeextract, 5 g/l NaCl. Als Antibiotikum wurde Ampicillin (Roth) in einer Konzentration von 500 mg/l nach dem Autoklavieren hinzugegeben.

M9-Medium: 2% Glucose, 30 g/l Na₂HPO₄, 15 g/l KH₂PO₄, 5 g/l NH₄Cl, 2,5 g/l NaCl, 0,015 g/l CaCl₂, 2,28 g/l MgSO₄, 0,05% Vitamin B1(nicht autoklaviert) 38,4 mg/l Arginin, 57,6 mg/l Isoleucin, 48 mg/l Phenylalanin, 57,6 mg/l Valin, 6 mg/ml Threonin, 50 mg/l Inositol, 40 mg/l Tryptophan, 15 mg/l Tyrosin, 60 mg/l Leucin, 4 mg/l Histidin.

Feste Nährböden wurden durch Zugabe von 1,6% Agar zu den Flüssigmedien hergestellt.

### 5. PCR (Polymerase Chain Reaction)

In einem Personal Cycler (Biometra, Göttingen) wurden PCR-Reaktionen mit dem PC-Long-Expand-Kit oder dem PCR-High-Fidelity-Kit der Firma Boehringer (Mannheim) in 50µl-Ansätzen durchgeführt. Eine Aufreinigung der Produkte erfolgte mit dem PCR-Purification-Kit der Firma Qiagen (Hilden) nach den Abgaben des Herstellers.

### 6. Transformation

### 6.1 Transformation von E.coli

### 6.1.1 Elektroporation

400 ml LB-Medium wurden 1:100 verdünnt aus einer ÜK angeimpft und bei 37°C bis zu einer OD₆₀₀ von 0,5-1 kultiviert. Nach 30 min. Inkubation auf Eis wurde die Kultur bei 4°C und 3000 rpm für 15 min. zentrifugiert. Die Zellen wurden 2x mit eiskaltem Wasser (1 Vol./1/4 Vol.) und 1x mit 1/50 Vol. 10%igem Glycerin gewaschen. Anschließend wurde das Zellpellet in 1,5 ml 10% Glycerin aufgenommen und 40 µl Aliquots bei -70°C aufbewahrt. Zur Transformation wurde ein Aliquot auf Eis aufgetaut und mit 0,01-1 µg Plasmid-DNA mit einem Gene Pulser (Bio Rad) bei 1,6 KV, 25 µF und 200 Ohm in einer 1 mm Küvette transformiert. Danach wurden die Zellen zur Erholung für 30-45 min. bei 37°C in 1 ml LB-Medium inkubiert und dann auf LB-amp Platten ausplattiert. Die Platten wurden bei 37°C inkubiert.

### 6.1.2 RbCl₂-Methode

Die Transformation von RbCl₂-Zellen erfolgte nach dem Protokoll von Hanahan (1985). Pro Transformation wurden 20-500 ng DNA mit 200 µl auf Eis aufgetauter kompetenter Zellen gemischt. Nach 20 min. Inkubation auf Eis, 45 sec. Hitzeschock (42°C) und erneuter 2 min. Inkubation auf Eis wurden die Zellen zur Erholung mit 1 ml LB-Medium versetzt und für 30-45 min bei 37°C inkubiert. Anschließend wurden die Zellen auf LB-amp-Platten ausplattiert, die dann über Nacht bei 37°C inkubiert werden.

### 6.2 Transformation von H. polymorpha durch Elektroporation

100 ml YPD wurden mit 5 ml einer dichtgewachsenen ÜK beimpft. Die Kultur wurde bei 37°C bis zu einer OD₆₀₀ von 0,8-1,2 geschüttelt (ca 3 Std.). Die Zellen wurden durch Zentrifugation bei 3000 rpm geemtet und in 20 ml KPᵢ-Puffer (50 mM/pH 7,5) resuspendiert. Nach Hinzufügen von 0,5 ml DTT und 15minütigem Schütteln bei 37°C wurden die Zellen bei 2500 rpm abzentrifugiert und 2x mit STM-Puffer (270 mM Saccharose, 10 mM TrisCl, 1 mM MgCl₂, pH 7,5) gewaschen. Danach wurden die Zellen in 0,25 ml STM-Puffer aufgenommen und 60 µl-Aliquots bei -70°C gelagert. Zur Transformation mit rDNA-Integrationsvektoren wurde die Plasmid-DNA zunächst mit *Xho*I oder *Sac*I linearisiert. 0,1-1 µg der linearisierten Plasmid-DNA wurden mit frische, auf Eis aufgetauten kompetenten Zellen gemischt, diese Ansätze dann in 2mm eine Küvette gegeben. Die Transformation erfolgte in einem Gene-Pulser (Bio-Rad, München) bei 2,0 kV, 25 µF und 200 Ohm. Anschließend wurden die Zellen zur Erholung in 1 ml YPD für 1 Std. bei 37°C inkubiert und dann auf Selektions-Medium ausplattiert. Nach 2-4 Tagen Inkubation bei 37°C wurden makroskopische Kolonien sichtbar.

### 7. Generierung von H. polymorpha-Stämmen

Zur Integration von Vektoren in die Hefe *H. polymorpha* wurden die aus der Transformation hervorgegangenen Uracil-prototrophen Klone dreimal nacheinander auf Selektionsmedium-Platten, einmal auf Vollmediumplatten, und dann nochmals auf Selektionsmedium-Platten übertragen (je nach 24 Std. Wachstum bei 37°C).

### 8. Dauerkultivierung von H. polymorpha

Für Stabilitätsstudien wurden 50 ml-Flüssigkulturen (YPD, 2% Glukose) über 27 Tage bei 37°C geschüttelt, dabei jeden Tag frisch überimpft. Die *H. polymorpha*-Kulturen hatten zum Zeitpunkt des Überimpfens und der Probenentnahme eine OD₆₀₀ zwischen 3 und 4, die Zellen waren also noch vor dem Eintritt in die stationäre Phase. Es wurden jeden zweiten Tag 2 ml-Glycerinkulturen (27%) angefertigt und bei -70°C aufbewahrt.

### 9. Präparation von Nukleinsäuren

### 9.1 Fragment-Isolierung aus Low-Melting-Gelen

Die DNA-Präperation aus Low-Melting Agarose-Gelen erfolgte mit Hilfe der Phenol/ Chloroform-Extraktion. Unter UV-Licht ausgeschnittene Gelblöcke mit DNA-Fragmenten wurden mit 130 µl Wasser versetzt und bis zum Schmelzen der Agarose bei 70°C erhitzt. Nach Zugabe von 150 µl Phenol und 50maligen Schütteln wurde die Probe zentrifugiert (2 min. bei 13000 rpm) und die untere Phenolphase verworfen. Nun wurden 150 µl Phenol/Chloroform (1:1 Mischung) auf die wässrige Phase gegeben, gemischt und erneut zentrifugiert. Wiederum wurde die untere Phase verworfen und die wässrige Phase zweimal mit Chloroform/Isoamylalkohol (1:24) gewaschen (je 150 µl). Anschließend wurde ein 1/10-Volumen NaAc (3 M, pH 8) und 1 ml 96%iger Ethanol hinzugefügt und die DNA durch 15 min. Inkubation bei -70°C gefällt. Nach Abzentrifugieren der DNA, einmaligem Waschen mit 70% Ethanol und anschließendem Trocknen wurde die DNA in 15-30 µl Wasser aufgenommen.

### 9.2. Fragment-Isolierung aus PCR-Ansätzen

Die Fragment-Isolierung aus PCR-Ansätzen und aus Low-Melting Gelen erfolgt mit Hilfe des PCR-Purification Kits (Quiagen, Hilden) entsprechend den Herstellerangaben.

### 9.3 Plasmid-Präparation E. coli (Mini-Präp)

Die Präparation von Plasmid-DNA aus *E. coli* erfolgte nach der von Maniatis *et al* (1982) beschriebenen Methode der alkalischen Lyse. 1,5 ml einer ÜK wurden bei 13000 rpm abzentrifugiert (2 min.). Das Zell-Pellet wurde in 100 µl P1 (50 mM Tris/HCl, 10 mM EDTA) resuspendiert. Die Lyse erfolgt durch Zugabe von 100 µl P2 (200 mM NaOH, 1% SDS). Proteine und sonstige Zellbestandteile wurden anschließend durch Zugabe von 100 µl P3 (2,55 M KOAc, pH 4,8) gefällt. Nach Zentrifiugieren für 10 min. bei 13000 rpm wurde die DNA aus dem Überstand mit dem doppelten Volumen 96%igem Ethanol gefällt. Nach Waschen und Trocknung wurde das Pellet in 20 µl Wasser aufgenommen. Der Reinheitsgrad dieser DNA war hinreichend für Restriktionsanalysen.

### 9.4. Plasmid-Präparation aus E. coli (Midi-Präp)

Für DNA-Präparationen höheren Reinheitsgrades und größerer Mengen wurde das Plasmid-Midi-Kit der Firma Quiagen nach Herstellerangaben verwendet.

### 9.5. Konzentrationsbestimmungen von Nukleinsäuren

Die Konzentration von DNA mit niedrigem Molekulargewicht (Oligonucleotide) erfolgte durch Absorptionsbestimmung bei 260 nm. Die Konzentration hochmolekularer DNA wurde durch Vergleich mit einem kalibrierten Mengenstandard (*KB*-Leiter, Pharmacia) im Agarosegel abgeschätzt.

### 9.6 Präparation chromosomaler DNA aus H. polymorpha

Zellen aus einer 2 ml ÜK wurden die bei 13 000 rpm geemtet und in 0,5 ml STEHp-Puffer (0,5 M Sorbitol; 0,1 M Tris/HCl; pH 8; 0,1 M EDTA) resuspendiert. Die Zellwand wurde durch Zugabe von 10 µl Zymolyase (2,5 mg/ml) und 1 µl β-Mercaptoethanol und Inkubation für 1 Std. bei 37°C entfernt. Unter dem Mikroskop wurde kontrolliert, ob die Zellen als Protoplasten vorliegen. Danach wurden die Protoplasten vorsichtig abzentrifugiert (5 min bei 3000 rpm), in TEHp-Puffer (50 mM Tris/HCl, pH 8, 20 mM EDTA) resuspendiert und mit 25 µl SDS (20%) versetzt. Die Lyse erfolgte durch Inkubation für 20 min. bei 65°C. Durch Zugabe von 200 µl KOAc und Inkubation auf Eis (30 min.) wurden Proteine und Zelltrümmer gefällt. Nach Zentrifugation bei 13 000 rpm für 10 min. wurde der Überstand durch Watte filtriert und die im Filtrat gelöste DNA mit 0,7 Volumen Isopropanol gefällt. Nach Waschen mit 70% Ethanol wurde das getrocknete DNA-Pellet in 20-30 µl Wasser aufgenommen.

### 9.7 Präparation chromosomaler DNA zur Gewinnung großer Fragmente

Zur Gewinnung von genomischer DNA von besserer Qualität wurde das in 9.6 beschriebene Standardprotokoll modifiziert. *H. polymorpha*-Zellen wurden aus 50 ml ÜK bei 3000 rpm geerntet. Die Zellen wurden in 30 ml STEHp-Puffer resuspendiert, abzentrifugiert und darauf in 1,5 ml STEHp aufgenommen. Zum Auflösen der Zellwand wurden 30 µl Zymolyase hinzugegeben. Die Protoplasten wurden mit 2 ml 5 M KOAc gemischt, das Lysat für 30 min. bei 13 000 rpm abzentrifugiert (4°C). Nun wurde der Überstand mit 2 ml 5 M NH₄OAc versetzt und die gelöste DNA mit 10 ml kaltem 96% Ethanol gefällt. Nach 5 min. Inkubation auf Eis wurde die DNA bei 3000 rpm 5 min. abzentrifugiert, mit 50%igem Ethanol gewaschen und in 2 ml TE (10 mM Tris/HCl, 0,1 mM EDTA, pH 7,5) aufgenommen. Diese gesamte Prozedur wurde in abnehmenden Volumina mehrfach wiederholt. Die DNA wurde schließlich in einem Volumen von 200 µl TE aufgenommen.

### 10. Southern Blot-Analyse

5 µg chromosomaler DNA (*H. polymorpha*) oder 0,5 µg Plasmid-DNA wurden über Nacht mit 1 U/µg der entsprechenden Restriktionsendonuclease behandelt. Die Auftrennung der DNA erfolgte in 0,6-0,8%-igen Agarose-Gelen. Durch lange Trennzeiten (2-12 Std. bei 200 mA) konnten auch relativ große (5-12 Kb) DNA-Fragmente unterschieden werden

Nach dem Photographieren des Geles wurde der DNA-Transfer auf eine Nylonmembran (Hybound M, Amersham) in einer Vakuum-Blot Apparatur (L*KB* 2016 Vacugene, Pharmacia) vorgenommen. Hierzu wurde zuerst für 15 min. mit Depurinierungslösung (0,25 M HCl) transferiert. Dieser zusätzliche Schritt ermöglicht den Transfer auch sehr großer Fragmente. Mit Denaturierungslösung (1,5 M NaCl, 0,5 N NaOH) und Neutralisierungspuffer (3 M NaAc, pH 5,5) wurde das Gel je 20 min. lang überschichtet. Abschließend wurde für 20 min. mit 20 x SSC Transferpuffer (3 M NaCl, 0,3 M NaCitrat, pH 7) überschichtet. Durch eine 2minütige Bestrahlung der Membran mit UV-Licht (254 nm) und anschließende 30minütige Inkubation bei 80°C wurde die DNA an die Nylonmembran fixiert.

Die Herstellung der Sonde, die Hybridisierung und der Nachweis des Hybrids erfolgten mit Hilfe einer enzymatischen Reaktion werden nach dem Protokoll des Dioxygenin-Labeling-Kit der Firma Boehringer (Mannheim). In einigen Fällen wurden die Southem-Blots mit einer zweiten Sonde hybridisiert und entwickelt. Dazu wurde die erste Sonde durch eine Abfolge von Waschschritten von der Membran entfernt (1 Std. in Dimethylformamid bei 60°C, dann Waschen in Lösung 1 (0,2 M NaOH, 0,1% SDS) und Lösung 2 (0,3 M NaCl, 0,03 M Citrat) für jeweils 10 min.).

### 11. X-Gal-Overlay-Assay - Nachweis von β-Galactosidase

Der Nachweis der β-Galactosidase-Aktivität erfolgte nach Suckow and Hollenberg (1998). Die zu testenden Stämme wurden für 4-6 Stunden in Selektionsmedium bei 37°C kultiviert. Ein 4 µl-Tropfen einer jeden Kultur wurde auf eine Selektionsplatte gegeben und über Nacht bei 37°C inkubiert. Die Platte wurde mit frischen Überschichtungsagar (0,5% Agarose, 0,5 M Na₂HPO₄/NaH₂PO₄ (pH 7); 0,2% SDS; 2% DMF (Dimethylformamid) 2 mg/ml X-gal (o-Nitrophenyl-β-D-Galactopyranoside) bei 70 °C überschichtet. Nach wenigen Minuten zeigten die Klone mit *lacZ*-Expression eine Blaufärbung.

### 12. Phytase-Nachweis

Aus 3 ml ÜK wurden die *H. polymorpha*-Zellen geerntet und in 200 µl YNB Medium und 1 ml 5%igem Glycerin aufgenommen. Nach 1-2 Tagen Wachstum wurde zuerst die OD₆₀₀ bestimmt. Die Zellen wurden dann abzentrifugiert und 25 µl des Überstandes weiterverwendet. Zu diesem Aliquot wurden 25 µl 5 M NaAc und 50 µl 4-Nitrophenylphosphat hinzugegeben. Der Ansatz wurde 30 min. bei 37°C inkubiert. Die enzymatische Umsetzung des Substrates wurde durch Zugabe von 100 µl 15%iger Trichloressigsäure abgestoppt. Nach Zugabe von 100 µl 1 M NaOH erschienen positive Kulturüberstandsproben intensiv gelb gefärbt. Die Gelbfärbung wurde durch OD₄₀₅-Messung im Photometer quantifiziert.

### 13. Immunologischer Nachweis von Insulin

Die Zellen wurden für 1-2 Tage in 2 ml Selektionsmedium (2% Glycerin) bei 37°C kultiviert. 4 µl-Aliquots dieser Kulturen wurden auf eine Selektionsplatte aufgetropft und diese über Nacht bei 37°C inkubiert. Danach wurde eine Nitrozellulose-Membran (Amersham Int., England) auf die Platte gelegt, dann erfolgte eine erneute Inkubation über Nacht bei 37°C. Die Membran wurde vorsichtig abgezogen, und alle an der Membran haftenden Zellen wurden mit Wasser sorgfältig abgespült. Nach dem Trocknen der Membran konnte das sezemierte Genprodukt auf den Filtern mit Insulin-Antikörpern immunologisch nachgewiesen werden. Der Nachweis erfolgte nach Standardmethoden mit Hilfe des NOVEX-Anti-Maus-Kits. Insulin-sezernierende Klone konnten mit dieser Methode eindeutig nachgewiesen werden.

### 14. Nachweis der GFP-Expression

Für diese Studie wurde eine speziell für die Translation in der Hefe *S. cerevisiae* optimierte Mutante des *wt*-GFP verwendet, das yeast enhanced green fluorescent protein3 (yEGFP). In *S. cerevisiae* zeigt dieses Protein eine stärkere Fluoreszenz als *wt*-GFP (Cormack *et al*., 1997). Auf dem Plasmid pM2GT befindet sich das Gen für yEGFP unter der Kontrolle des *TPS1*-Promotors aus *H. polymorpha*. Es konnte gezeigt werden, daß yEGFP in *H. polymorpha* zu einer mit *S. cerevisiae* vergleichbaren Fluoreszenz führt. In dieser Studie wurde die GFP-Expression qualitativ analysiert. Hierfür wurden die Stämme in Doppelproben für 12 Std. bei 37°C in YNB-Medium (2 ml-Kulturen) herangezogen und unter dem Fluoreszenz-Mikroskop auf grünes Leuchten untersucht.

### Beispiel 1

### Gewinnung und Charakterisierung eines rDNA-Fragmentes für die Konstruktion eines rDNA-Integrationsvektors

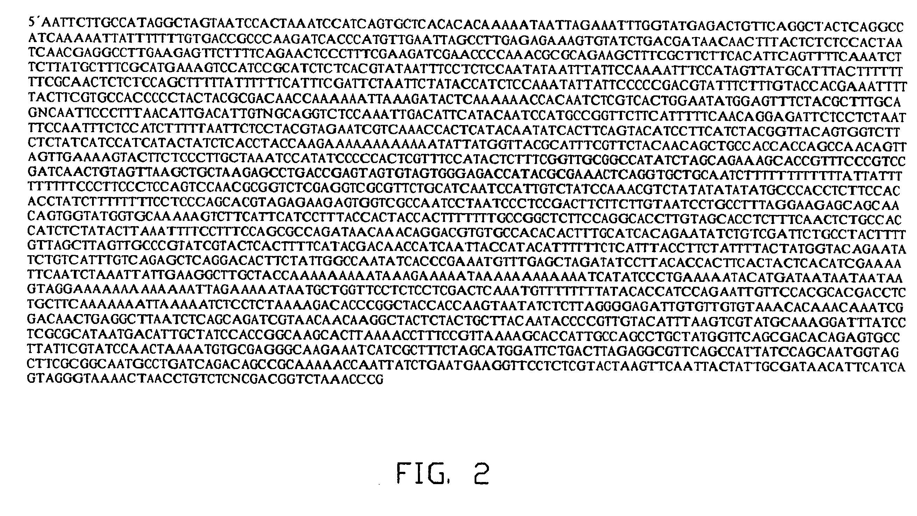

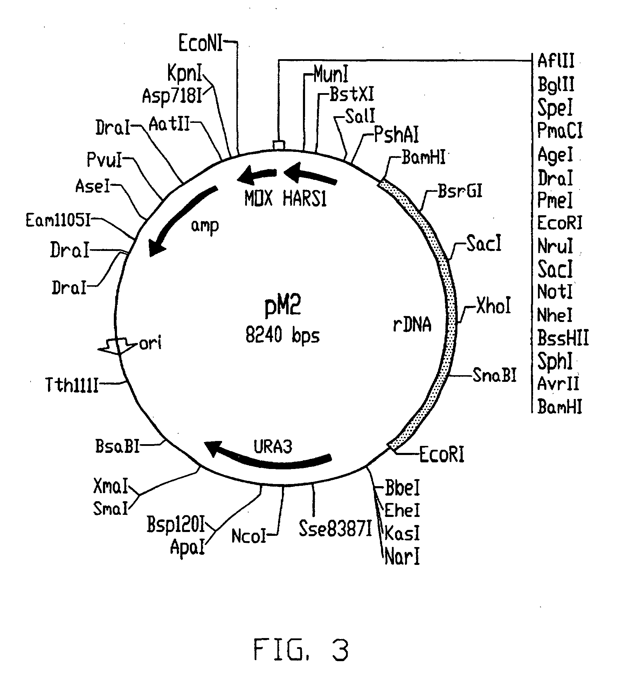

Voraussetzung für die Integration eines Vektors in die rDNA von *H. polymorpha* ist die Anwesenheit einer Sequenz, die zu diesem Bereich homolog ist. Dazu wurde ein 2,4 Kb langes rDNA-Fragment aus dem rDNA-Bereich von *H. polymorpha* festgelegt. Im folgenden wird diese 2,4 Kb lange Sequenz (SEQ ID NO 1) (Figur 2) als Integrationssequenz bezeichnet. Für die Klonierung dieses Fragments aus der ribosomalen DNA von *H. polymorpha* wurde mit Hilfe einer PCR-Reaktion mit den Primern A (5'-ACG GGA TCC GGG TTT AGA CCG TCG TGA GAC AGG-3') und B (5'-CCT GGA TCC TAC CAT CGA AAG TTG ATA GGG-3') zunächst ein 3 Kb langes Fragment aus der chromosomalen DNA von *H. polymorpha* amplifiziert. Die Festlegung der Primer erfolgte nach Sequenzen der 18S- und 25S-rDNA aus *S. cerevisiae* mit flankierenden *Bam*HI-Erkennungssequenzen. Das Amplifikat wurde später durch *Eco*RI/*Bam*HI-Restriktion (das Fragment enthält eine interne *Eco*RI-Erkennungssequenz) auf 2,4 Kb verkürzt. Das 2,4 Kb lange Fragment wurde in den Vektor pM1 kloniert und vollständig sequenziert. Es entstand der Basisvektor pM2 (Figur 3), aus dem alle in dieser Anmeldung beschriebenen Plasmide hervorgegangen sind.

Durch Vergleich mit bekannten Sequenzen aus der rDNA-Einheit von *S. cerevisiae* konnte das 5S-rRNA-Gen innerhalb der klonierten Sequenz nachgewiesen werden. Die 5S-DNA aus *H. polymorpha* besitzt eine 96%ige Homologie zu dem entsprechenden Gen von *S. cerevisiae*. Das 5S-Gen befindet sich an Position 958-1079 der Integrationssequenz, gezählt von der flankierenden *Eco*RI-Schnittstelle. Mit diesem Befund und der Kenntnis über die Isolierung der Integrationssequenz konnte die Position des klonierten rDNA-Fragmentes in einem rDNA-Repeat von *H. polymorpha* festgelegt werden und eine gleichartige Organisation der rDNA-Sequenzen wie in *S. cerevisiae* angenommen werden. Teilbereiche des ITS2 und der 18S-Sequenz gehen bei der Verkürzung verloren. Die Position des klonierten Fragmentes in der rDNA-Einheit von *H. polymorpha* wird in Figur 1 (C) gezeigt. Seine vollständige Sequenz ist in der nachfolgenden Figur 2 dokumentiert. Die grau schattierte Sequenz zeigt die Lage der 121 bp langen 5S-rDNA.

### Beispiel 2

### Herstellung der Basis-rDNA-Integrationsvektoren pM2 und pM2Δ

Das im vorigen Beispiel beschriebene 2.4 Kb *Eco*RI/*Bam*HI-rDNA Fragment wurde in einen *E. coli* / *H. polymorpha* Shuttle-Vektor pM1 eingebracht, der die folgenden Elemente enthält: *HARS1* und *MOX*-Terminator aus *H. polymorpha; ori* and *amp*^{*R*} aus *E. coli*. Zwischen *HARS1* und *MOX*-Terminator ist eine Polyklonierungstelle für die Aufnahme heterologer Promotor/Genfusionen positioniert. Einige Beispiele der resultierenden Integrations/Expressionsvektoren sind in den Beispielen 3 und 4 angegeben. Die integrierte rDNA enhält eine *Brs*GI, *Sac*I, *Sna*I und eine *Xho*I-Erkennungssequenz zur Linearisierung des resultierenden Vektors M2. Der Vektor ist in Figur 2 dargestellt.

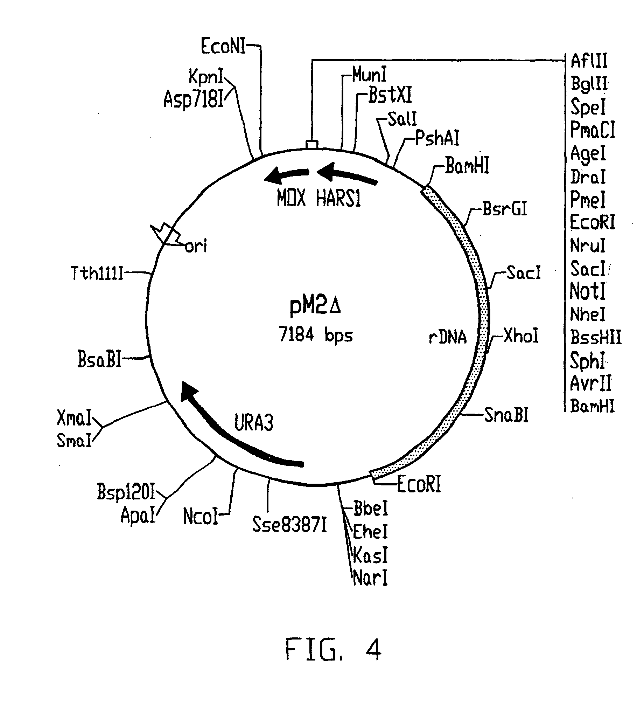

Zur Generierung des Grundvektors pM2Δ wurde der Vektor pM2 mit *Dra*I/*Aat*II verdaut, und das resultierende 8,7 Kb-Fragment nach Entfernen des 3'-Überhanges des *Aat*IIkohäsiven Endes mit T4-Polymerase religiert. Es entsteht ein verkürzter Shuttle-Vektor ohne *amp*^{*R*}. Die Propagierung dieses Vektors und seiner Derivate wurde in dem *E.coli*-Stamm MC1066 durchgeführt, der Uracil-auxotroph ist, und somit über das *Ura3*-Gen auf Uracil-Prototrophie selektioniert werden konnte. Der Grundvektor M2Δ ist in Figur 4 dargestellt.

### Beispiel 3

### Integrations-/Expressionsvektor für die Expression von lacZ

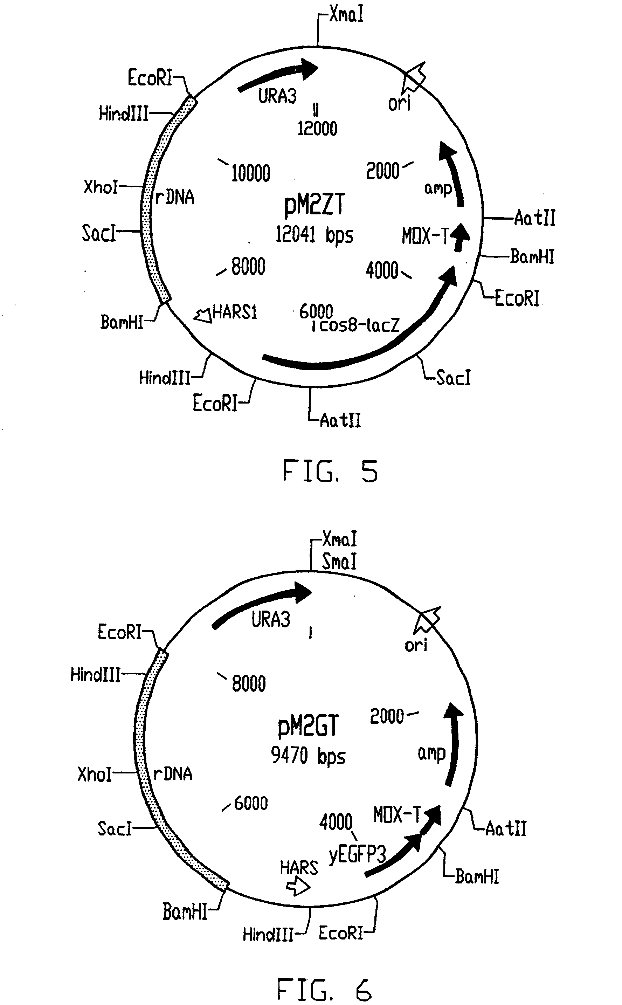

Der in Figur 5 dargestellte Vektor pM2ZT wurde durch Integration einer Expressionskassette für das bakterielle *lacZ*-Gen in die Polyklonierungsstelle von pM2 generiert. Als Promotorelement wurde der *TPS1*-Promotor aus *H. polymorpha* verwandt (pM2ZT), in Beispiel 4 wurde durch Integration eines geeigneten Fragmentes in pM2 eine Expressionskassette für GFP generiert. Als Promotorelement wurde der *TPS1*-Promotor aus *H. polymorpha* verwendet.

### Beispiel 4

### Integrations-/Expressionsvektor für die Expression von GPF

Der in Figur 6 dargestellte Vektor pM2GT wurde durch Integration einer Expressionskassette für *GFP* in die Polyklonierungsstelie von pM2 generiert. Als Promotorelement wurde der *TPS1*-Promotor aus *H. polymorpha* verwendet.

### Beispiel 5

### Integrations-/Expressionsvektoren für die Expression eines Insulin- und eines Phytase-Gens unter der Kontrolle des TPS1- und des FMD-Promotors

In dem folgenden Beispiel sind weitere Expressionsvektoren beschrieben. Sie sind Derivate von pM2 und pM2Δ und wurden, wie für die Beispiele 3 und 4 gezeigt, durch Insertion eines geeigneten Fragmentes in die Polyklonierungsstelle der beiden Grundvektoren generiert. Sie enthalten Expressionskassetten für die Synthese von Insulin und Phytase. Neben *TPS1* wurde der *FMD*-Promotor als Kontrollelement für die heterologe Genexpression eingesetzt. Es wurden folgende Vektoren generiert:
- pB11: Derivat von pM2 mit heterologer Expressionskassette für Insulin (*FMD*-Promotor vor einem Insulin-Gen), *H. polymorpha* -rDNA-Integrationssequenz, *HARS1, URA3 (S. cerevisiae), MOX*-Terminator, *ori, amp*^{*R*}.
- pB14: Wie pB11, jedoch mit synthetischem Phytase-Gen anstelle des Insulin-Gens.
- pB14X: M2Δ-Derivat mit heterologer Expressionskassette. Wie pB14, jedoch ohne *amp*^{*R*}

### Beispiel 6

### Generierung rekombinanter H. polymorpha-Stämme

Die Transformation von RB11 mit rDNA-Integrationsvektoren verlief nach einem generellen Prinzip, das für alle hier gezeigten Transformationen gilt. Durch die Restriktion mit einem Enzym, für das nur eine singuläre Schnittstelle innerhalb der Integrationssequenz vorliegt (z.B. *Sac*I oder *Xho*I), wurde die Integrationssequenz in zwei endständige Teilsequenzen geteilt, die mit entsprechenden Sequenzen auf der rDNA-Einheit homolog rekombinieren können. Für nicht-tinearisierte Plasmide konnte bisher keine Integration in die rDNA nachgewiesen werden. Die rDNA-Integrationsvektoren enthielten als Selektionsmarkergen das intakte *URA3*-Gen aus *S. cerevisiae*. Die Nutzung eines solchen intakten Genes führt, wie in den nachfolgenden Beispielen gezeigt wird, zu mitotisch stabilen Stämmen, in denen die rekombinante integrierte DNA in 40 - 50 Kopien vorliegen kann.

Die Transformation wurde durch Elektroporation durchgeführt (s. Material und Methoden). Die Integration in das Genom erfolgt durch Wachstum unter Selektionsdruck über 30 Generationen (YNB-Platten oder Medien, die kein Uracil enthalten). Bei der herkömmlichen Methode zur Generierung rekombinanter *H. polymorpha*-Stämme mit zirkulären Plasmiden sind mindestens 80 Generationen Wachstum auf selektiven Medien notwendig (Gatzke et al. 1995). Damit steht bei Nutzung von rDNA-Integrationsvektoren eine gegenüber den etablierten Protokollen einfachere und schnellere Methode zur Generierung rekombinanter *H. polymorpha*-Stämme zur Verfügung.

### Beispiel 7

### Transformation mit einem Vektor - Generierung rekombinanter Stämme mit pB14 und deren Charakterisierung

Die rDNA in *H. polymorpha* besteht aus ca. 40 aufeinander folgenden Einheiten, die identisch aufgebaut sind (Beispiel 1). Die Länge einer Einheit beträgt ca. 8 Kb. Diese Länge kann durch Hybrisationsexperimente mit rDNA-Sonden ermittelt werden, falls in Restriktionen Enzyme genutzt werden, die nur einmal in einem rDNA-Repeat schneiden. Eines dieser Enzyme ist *Bgl*II. Die Integration ist in eine oder mehrere dieser rDNA-Einheiten möglich.

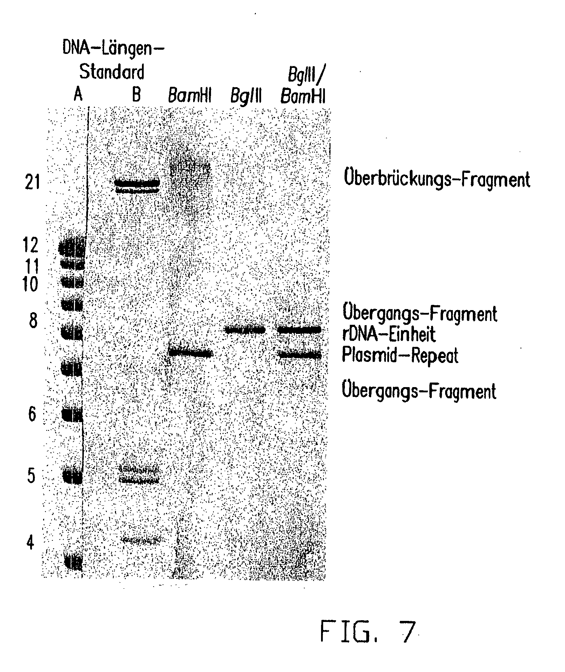

Der Uracil-auxotrophe Stamm RB11 wurde mit pB14 nach der oben beschriebenen Methode transformiert. Die Linearisierung erfolgte durch *Sac*I. Dadurch wurde das rDNA-Fragment des Vektors auf etwa zwei gleich lange Randsequenzen verteilt. Von den generierten rekombinanten Stämmen wird im folgenden ein repräsentatives Beispiel dokumentiert. Der Struktumachweis der in die rDNA integrierten heterologen DNA basiert auf folgenden Vorüberlegungen: Wird ein Enzym verwendet, das im Vektor, aber nicht in der rDNA schneidet (*Bam*HI), und wird dieses zusammen mit *Bgl*II verwendet (schneidet in der rDNA-Einheit, aber nicht im Vektor), so müßten sich Fragmentgrößen identifizieren lassen, die den Übergang von einer rDNA-Repetitionseinheit in eine Vektorkopie anzeigen. Für den Fall, daß mehrere Kopien des Vektors an verschiedenen Positionen integriert sind, müßten sich auch sehr lange Überbrückungsfragmente nachweisen lassen, da *Bam*HI nur im Vektor und *Bgl*II nur in der rDNA schneidet. Figur 7 zeigt diese Analyse.

Die mit den angegebenen Restriktionsenzymen geschnittene DNA hybridisiert mit der Sonde, die aus der Markierungsreaktion der Integrationssequenz hervorgegangen ist (siehe Material und Methoden). In der Spur mit der *Bam*HI-behandelten DNA ist ein starkes Signal bei 7,4 Kb zu erkennen. Dies entspricht dem Vektor-Fragment. In dieser Spur wird noch ein schwach erkennbares Signal bei über 21 Kb sichtbar. Dieses Fragment wird als Überbrückungsfragment interpretiert. In der Spur mit *Bgl*II-behandelter DNA ist ein starkes Signal bei 8 Kb erkennbar, was der Länge einer rDNA-Einheit entspricht. In der Spur mit dem Doppelverdau sind sowohl die rDNA- als auch die Vektor-Signale erkennbar. Bei 8,8 Kb und bei 6,7 Kb sind schwache Signale zu sehen, die Übergangsfragmente von rDNA in Plasmid-DNA repräsentieren. In der ersten Spur wurde als DNA-Längenstandard die Kilobasenleiter, in der zweiten Spur *Eco*RI/*Hind*III-verdaute λ DNA aufgetragen.

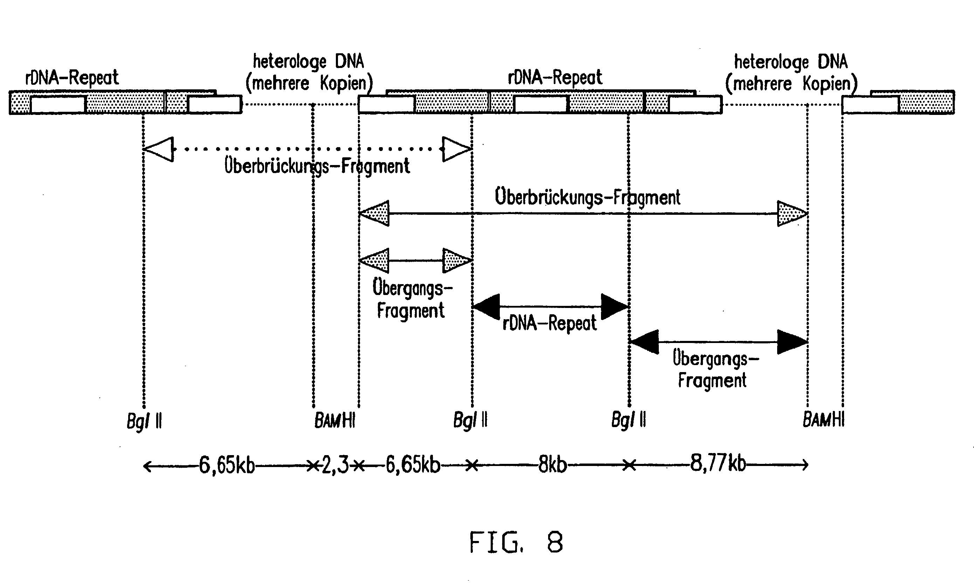

Daraus ergibt sich folgender Befund: Die heterologe DNA liegt in mindestens zwei Clustern vor. Die Zahl der Plasmid-Kopienzahl pro Cluster muß erheblich mehr als zwei betragen. Zwischen den Plasmid-Clustem liegt mindestens eine intakte rDNA-Einheit. Figur 8 zeigt die aus der Analyse abgeleitete Struktur.

### Beispiel 8

### Simultane Integration von drei Vektoren in den rDNA-Bereich des Genoms von H. polymorpha und Charakterisierung der resultierenden Stämme

Es sollte die Möglichkeit untersucht werden, mehrere Vektoren mit identischem Basisdesign gleichzeitig in unterschiedlichen Clustern in die rDNA zu integrieren. Dadurch ergibt sich die Option, mit geringem Aufwand rekombinante Stämme für die Koproduktion von zwei oder mehr Proteinen bereitzustellen. RB11 wurde deshalb mit den drei Vektoren pM2, pB11 und pB14 (siehe Beispiele 2 und 5) transformiert und die 40 entstandenen rekombinanten Stämmen auf das Vorhandensein integrierter Vektoren untersucht. Die Vektoren pB11 und pB14 wurden durch *Sac*I-Restriktion, pM2 durch *Xho*I-Restriktion innerhalb der Integrationssequenz linearisiert und in einem Transformations-Ansatz in äquimolaren Mengen eingesetzt. Die drei Vektoren besitzen übereinstimmend die 2,4 Kb lange Integrationssequenz zur homologen Rekombination in den rDNA-Bereich des Genoms von *H. polymorpha* und das *URA3*-Gen *aus S. cerevisiae* (s. Beispiel 1).

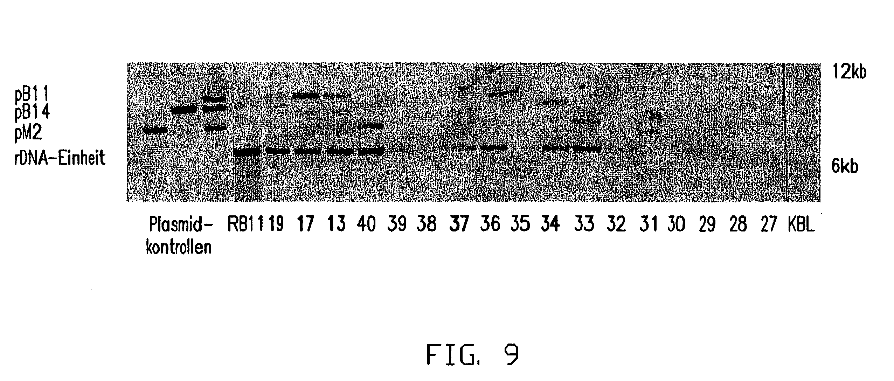

Die chromosomale DNA der aus dem Ansatz hervorgegangenen rekombinanten Stämme wurde mit *Aat*II (schneidet in jedem der drei Vektoren nur einmal) geschnitten, die Fragmente in einem Agarosegel aufgetrennt und einer Southern-Blot Analyse unterzogen. Als Sonde wurde die Digoxygenin-markierte Integrationssequenz eingesetzt. Durch die Restriktion der heterologen DNA mit einem "single cutter" wurde gewährleistet, das für die Vektoren Fragmente entstehen, die klar zugeordnet werden können, und die mit der Sonde hybridisieren. Figur 9 zeigt eine Auswahl der analysierten Stämme.

Die fett gedruckten Ziffern kennzeichnen die Stämme, die zwei oder drei verschiedene Vektoren gleichzeitig integriert tragen. Bei den Stämmen RB3T-13, RB3T-17 und RB3T-37 sind auf der Höhe von pM2 und pB14 Signale erkennbar, sie tragen beide Vektoren genomisch integriert. RB3T-19 zeigt für alle drei Vektoren ein Signal. Durch die Restriktion mit *Aat*II erscheint bei der Kontrolle RB11 ein Signal bei 6,5 Kb, welches die rDNA-Einheit repräsentiert und auch bei den rekombinanten Stämmen in Erscheinung tritt.

Stamm RB3T-34 zeigt für pB14 eine starke, und für pM2 eine schwächere Bande. Bei diesem rekombinanten Stamm kam es zu einer anderen Kombination der Kointegration. in der letzten Spur ist der DNA-Längenstandard aufgetragen (KBL).

Der Vektor pM2 konnte in 20 Stämmen (No.1-13,17,19,22,33-40), pB14 in sieben Stämmen (No.4,6,11,19,21,22,34) und pB11 in sieben Stämmen (No.4,6,11,19,21,22,34) nachgewiesen werden. Es konnten vier rekombinante Stämmen identifiziert werden, in denen zwei verschiedene Vektoren integriert waren (No. 17, 13 und 37 mit pM2 und pB11; No. 34 mit pM2 und pB14). Ein Stamm (No. 19) enthält alle drei Vektoren. Die rekombinanten Stämme wurden nach folgender Nomenklatur bezeichnet: RB für *H. polymorpha*-Stamm RB11; 3T für Transformation von RB11 mit 3 verschiedenen Vektoren.

### Beispiel 9

### Kopienzahl-Bestimmung der integrierten Vektoren

Für alle generierten rekombinanten Stämme des in Beispiel 8 beschriebenen Transformationsansatzes wurden Kopienzahlen zwischen 40 und 50 nachgewiesen. Sie entspricht demnach in etwa der Gesamtzahl der rDNA-Repetitionseinheiten in der rDNA bei *H. polymorpha*. Bei der Kointegration unterschiedlicher Vektoren teilt sich diese Gesamtzahl auf die Kopienzahlen der einzelnen Vektoren auf. Als Beispiel für die Kopienzahlbestimmung wurde der Stamm RB3T-19 mit drei verschiedenen integrierten Vektoren gewählt. Für die Hybridisation wurde eine *MOX*-Terminator Sonde verwendet. Die Sequenz für den *MOX*-Terminator kommt nur einmal im Genom von *H. polymorpha* vor und findet sich auf allen drei Vektoren. Somit wurde eine Sonde generiert, die sowohl mit den Vektorfragmenten, als auch mit der genuinen "single copy" *MOX*-Terminator-Sequenz des Wirtes hybridisiert. Die chromosomale DNA von RB3T-19 wurde mit *Aat*II geschnitten und analysiert. Die geschnittene DNA wurde in definierten Verdünnungsstufen aufgetragen. Die Stärke des Hybridisationssignals der heterologen Sequenz in den Verdünnungen kann mit der des genuinen Gens verglichen werden. Alle drei Signale für die integrierten Vektoren sind mit abnehmender Intensität in den jeweiligen Verdünnungen zu erkennen. Das Signal für das genuine *MOX*-Terminator-Fragment ist nur schwach zu erkennen. Die Signale für pB11 und pM2 in der 1/20-Verdünnung entsprechen ungefähr dem *MOX*-Terminator-Signal. Für pB14 zeigt sich in der 1/5-Verdünnung ein vergleichbar starkes Signal. Hieraus ergibt sich die im Text erwähnte Kopienzahl von jeweils 20 für die Vektoren pM2 und pB11, und fünf Kopien für pB14. Für die co-integrierten Vektoren pB11 und pM2 konnte eine Kopienzahl von jeweils 20 ermittelt werden. Für pB14 ergibt sich eine Kopienzahl von 5. Somit wird eine Gesamtkopienzahl für alle drei kointegrierten Vektoren von 45 erreicht.

### Beispiel 10

### Mitotische Stabilität rekombinanter H. polymorpha-Stämme

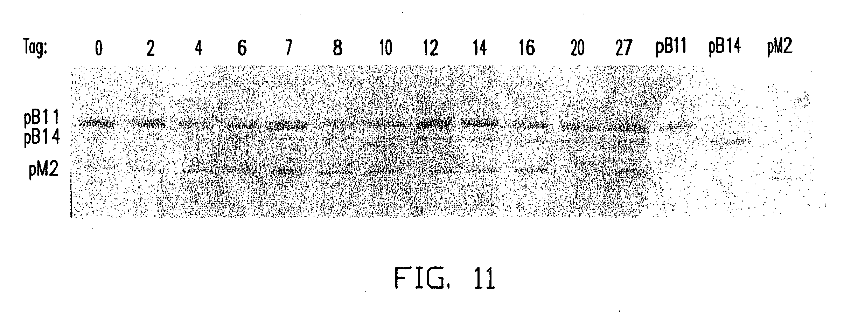

Die mitotische Stabilität rekombinanter Stämme, die durch Transformation von *H. polymorpha* mit drei rDNA-Integrationsvektoren generiert worden waren, wurde beispielhaft an den Stämmen RB3T-13, RB3T-17, RB3T-19 und RB3T-37 untersucht. Dazu wurden die Stämme über 27 Tage in einer 50 ml Flüssigkultur in nicht-selektivem (YPD) Medium kultiviert. In regelmäßigen Abständen wurden Proben entnommen und gesammelt. Die genomische DNA der gesammelten Proben wurde präpariert und nach der Restriktion mit *Aat*II einer *Southern Blot*-Analyse unterzogen. Als Sonde wurde das *Aat*II/*Bgl*II *MOX*-Terminator-Fragment aus dem Vektor pM2 verwendet. Für alle vier rekombinanten Stämme konnte die mitotische Stabilität in *Southern* Blot-Analysen nachgewiesen werden. Figur 11 zeigt das Ergebnis der *Southern Blot*-Analysen exemplarisch für den Stamm RB3T-19. Die unveränderte Intensität aller Signale für die Vektor-Fragmente über den gesamten Zeitraum zeigt die mitotische Stabilität des analysierten Stamms.

### Beispiel 11

### Transformation von RB11 mit fünf unterschiedlichen Vektoren

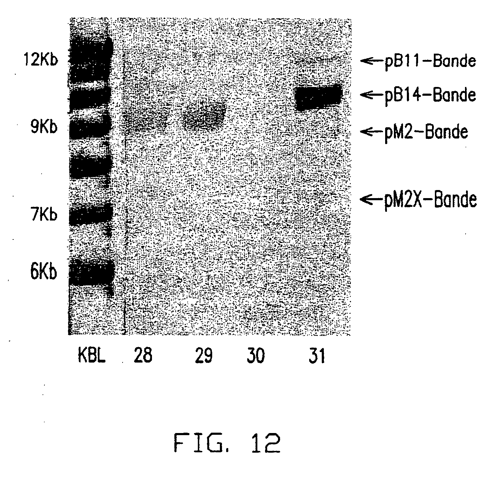

Ziel dieses Experimentes war es, die Zahl der gleichzeitig bei einer Transformation genutzten Vektoren zu steigern und so eine Obergrenze für eine Kointegration zu bestimmen. RB11 wurde mit den Vektoren pB11 (10,7 Kb), pB14 (9,7 Kb), pB14X (8,7 Kb), pM2 (8,2 Kb) und pM2X (6,7 Kb) kotransformiert. Die rekombinanten Stämme wurden in *Southern Blot*-Analysen überprüft. 80 rekombinante Stämme wurden analysiert. Die genomische DNA der rekombinanten Stämme wurde mit *Nco*I geschnitten, für das ebenfalls in allen Vektoren nur eine einzige Restriktionsstelle existiert. Als Sonde wurde die Digoxygenin-markierte *MOX*-Terminator Sequenz eingesetzt. In 66 Fällen war nur eine Vektor-Spezies integriert, in sieben Fällen waren zwei, in sechs Fällen drei und in einem Fall vier der insgesamt fünf Vektoren. Ein Stamm, der alle fünf Vektoren genomisch integriert trug, konnte unter den 80 analysierten rekombinanten Stämmen nicht entdeckt werden. Die Figur 12 zeigt die *Southern Blot*-Analyse für vier ausgewählte Stämme, wobei drei dieser Stämme Sequenzen von mindestens einem Integrationsvektor enthalten. Die chromosomale DNA wurde mit *Xho*I geschnitten. Die Analyse der geschnittenen DNA erfolgte wie in Figur 11. Stamm RB5T-31 enthält vier unterschiedliche Vektor-Spezies (pM2, pM2X, pB14X und pB11). Das Signal für pB14 bei 9,7 Kb fällt besonders stark aus. Dies spricht für eine im Vergleich zu den anderen Vektoren wesentlich höhere Kopienzahl.

### Beispiele 12 - 15

### Expression verschiedener Reportergene in rekombinanten H. polymorpha-Stämmen, die durch Integration von mindestens einem rDNA-Vektor generiert wurden

Nachdem in den vorausgegangenen Beispielen gezeigt wurde, daß eine Kointegration mehrerer verschiedener Vektoren in die rDNA von *H. polymorpha* zu mitotisch stabilen rekombinanten Stämmen führt, wird in den folgenden Beispielen die Exprimierbarkeit von vier verschiedenen Reporter-Genen nachgewiesen. Als Reporter-Gene wurden *GFP, lac*Z, ein Gen für eine Insulin-Variante und ein *Phytase*-Gen gewählt, weil die Detektion der Genprodukte leicht durchzuführen ist. Die Reporter-Gene sind auf den Vektoren pM2GT (*GFP*) pM2TZ (*lacZ*), pB11 (Insulin) und pB14 (Phytase) enthalten. Die vier Vektoren wurden jeweils durch *Sac*I-Restriktion innerhalb der Integrationssequenz linearisiert und in äquimolaren Mengen in einen Kotransformationsansatz gegeben. 132 resultierende rekombinante Stämme wurden auf das Vorhandensein der Genprodukte analysiert.

### Beispiel 12

### Expression von lacZ unter der Kontrolle des TPS1-Promotors

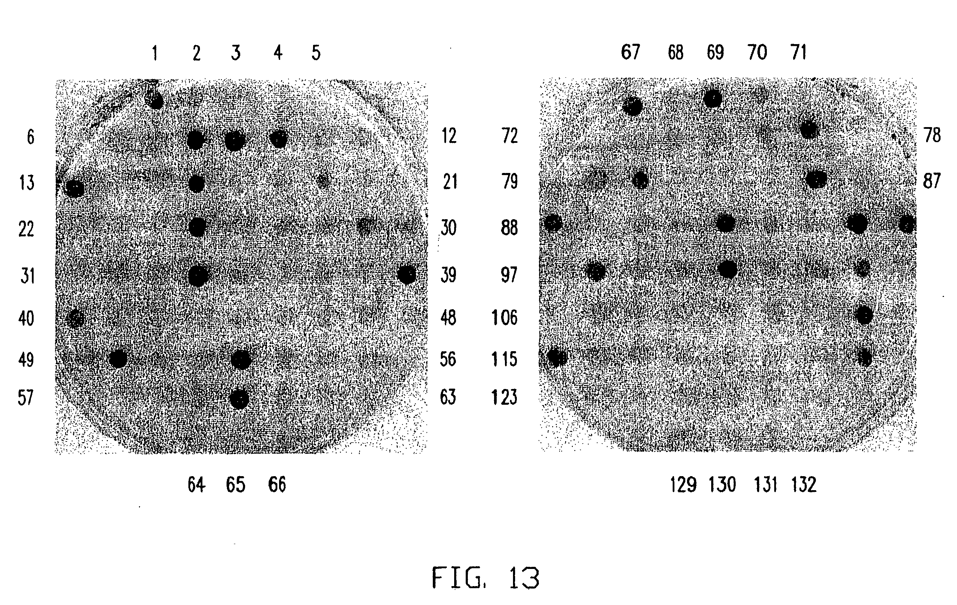

Das *lacZ*-Gen liegt bei dem hier verwendeten Vektor pM2ZT hinter dem *TPS1*-Promotor aus *H. polymorpha*. Dieser Promotor wird durch höhere Kultivierungstemperaturen aktiviert. Bei Wachstum bei 40°C auf YNB-Platten mit Glucose als einziger Kohlenstoffquelle wird der Promotor hinreichend aktiviert. Die Zellen wurden 6 Std. lang bei 40°C in Selektionsmedium kultiviert und dann jeweils 5 µl der Kulturen auf Selektions-Platten getropft. Nach 24 Std. Wachstum wurden die Kolonien mit Hilfe eines X-Gal Overlay-Assay (siehe Material und Methoden) auf β-Galactosidase-Aktivität untersucht. Nach 5stündiger Inkubation wurde das Ergebnis dokumentiert (Figur 13). Eine Schwärzung der Kolonie entspricht einer starken β-Galactosidase Aktivität. Graue Kolonien zeigen mittlere oder schwache Aktivität. 61 der 132 rekombinanten Stämme zeigten β-Galaktosidase-Aktivität. Dieses Ergebnis wurde in einem Wiederholungsexperiment bestätigt.

### Beispiel 13

### Expression des Insulin-Gens unter der Kontrolle des FMD-Promotors

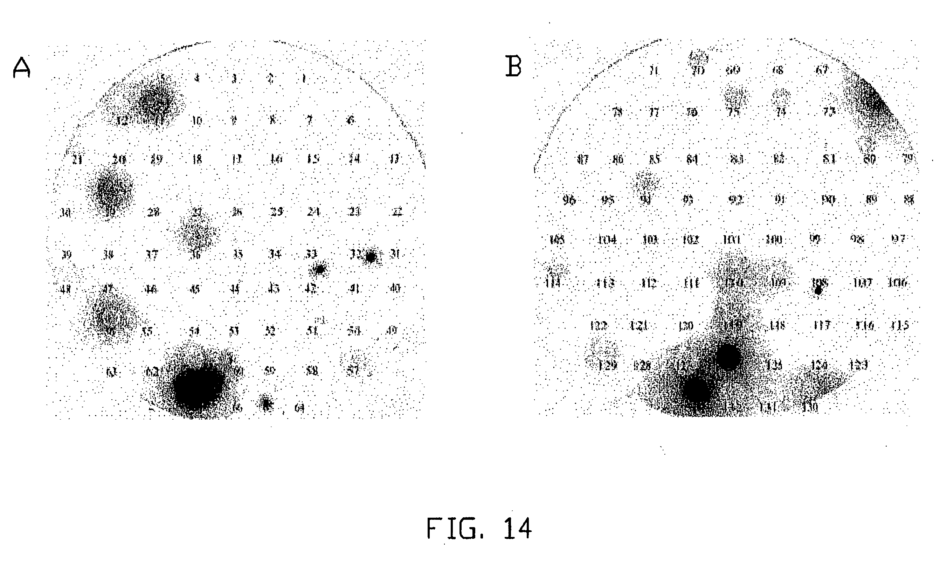

Der eingesetzte Vektor pB14 trägt eine Expressionskassette mit dem Gen für eine Insulin-Variante unter der Kontrolle des *FMD*-Promotors. Hierfür wurden die Zellen in einer Vorkultur (YNB, 5% Glycerin) 6 Std. bei 37°C inkubiert und je 5 µl davon auf YNB-Platten (2% Glycerin) aufgetropft. Als Kohlenstoffquelle wurde in diesem Fall Glycerin verwendet, da das Insulin-Gen unter der Kontrolle des *FMD*-Promotors steht. Dieser Promotor wird durch Glucose reprimiert und durch Glycerin dereprimiert. Zur Identifikation der Insulinproduzierenden Stämme wurde ein immunologischer Filtertest verwendet (siehe Material und Methoden). Nach Entwicklung des Filters zeigen sich Färbungen an den Positionen Insulin-positiver Kolonien (siehe Figur 14). Die Färbung des Filters ist proportional zur sezemierten Insulin-Menge. Bei 17 der 132 rekombinanten Stämme konnte Sekretion von Insulin gezeigt werden. Auf Filter A in Figur 14 zeigen vier Stämme eine mäßig starkes Signal. Stamm Nr. 51 und Stamm Nr. 56 zeigen eine schwache Färbung. Stamm Nr. 126 zeigt ein mit der Positivkontrolle vergleichbar starkes Signal. Die Stämme Nr. 80, 94 und 114 zeigen schwache Färbungen.

### Beispiel 14

### FMD-Promotor-kontrollierte Expression eines Phytase-Gens

Der Vektor pB11 trägt das Gen für die con-Phytase unter der Kontrolle des *FMD*-Promotors. Bei dieser Phytase handelt es sich um ein Mutein (Hoffman La Roche, Basel). Zur Bestimmung der Phytase-Aktivität wurden die Stämme in 3 ml-Übernachtkulturen (YPD, 37°C) herangezogen, und im Kulturüberstand wurde anschließend die Phytase-Aktivität photometrisch bestimmt (siehe Material und Methoden). 20 Stämme zeigten Phytase-Aktivität.

### Beispiel 15

### TPS1-Promotor kontrollierte Expression des GFP-Gens.

Der Vektor pM2GT trägt das *yEGFP*-Gen unter der Kontrolle des *TPS1*-Promotors aus *H. polymorpha*. Ist das Reporter-Protein in der rekombinanten Zelle in ausreichenden Mengen akkumuliert, so erfolgt nach Anregung mit Licht der Wellenlänge von 395 nm eine Fluoreszenz mit dem Emmissionsmaximum bei 510 nm (Morise *et al*., 1974); die Zellen leuchten intensiv grün (siehe Material und Methoden). Die Stämme wurden für 24 Stunden in YNB-Medium bei 37°C inkubiert. Anschließend wurden unter dem Fluoreszenz-Mikroskop Stämme mit GFP-Signal identifiziert. Das Experiment wurde insgesamt dreimal wiederholt und solche Stämme als positiv determiniert, die in allen drei Analysen ein Signal zeigten. Bei 36 der 132 Stämme wurde GFP nachgewiesen.

### Beispiel 16

### Koexpression von unterschiedlichen Reportergenen

Durch Cotransformation mit vier verschiedenen Vektoren wurden die in den Beispielen 12 bis 15 dokumentierten 132 rekombinante Stämme generiert. Alle Stämme wurden auf die Expression der oben beschriebenen Reporter-Gene hin untersucht. 28 zeigten keine Expression. Bei den verbleibenden *H. polymorpha*-Stämmen scheint eine zufällige Verteilung der Vektoren stattgefunden zu haben. 52 Stämme weisen eine Expression von *lacZ* auf, das hinter dem in *H. polymorpha* sehr starken *TPS1*-Promotor liegt. In 36 Fällen wurde das *GFP*-Gen exprimiert. Auf dem verwendeten Vektor liegt das *GFP*-Gen ebenfalls hinter dem *TPS1*-Promotor. Bei 20 Stämme wurde eine Expression des Phytase-Gens, in 17 eine Expression des Insulin-Gens beobachtet. 24 Stämme weisen eine Koexpression von zwei Reportergenen auf. In diesen Fällen wurde das Auftreten aller Kombinationen der vier Reporter-Gene beobachtet. Es wurden zwei rekombinante Stämme entdeckt, die eine Koexpression von drei Reportergenen zeigen: In Stamm RB4T-75 konnte eine Kombination von GFP, *lacZ* und Insulin, in Stamm RB4T-81 *lac*Z, Phytase und Insulin nachgewiesen werden.

Tabelle 1 zeigt eine Ubersicht der Expression von vier Reporter-Genen in rekombinanten *H. polymorpha* Stämmen. 132 rekombinante Stämme wurden analysiert. Die Stämme, die eine Koexpression mehrerer Reporter Gene zeigen, sind grau unterlegt, Stämme mit der Koexpression von drei Genen durch Rahmung hervorgehoben (+= starke Expression, O= mittlere Expression, - =schwache Expression).

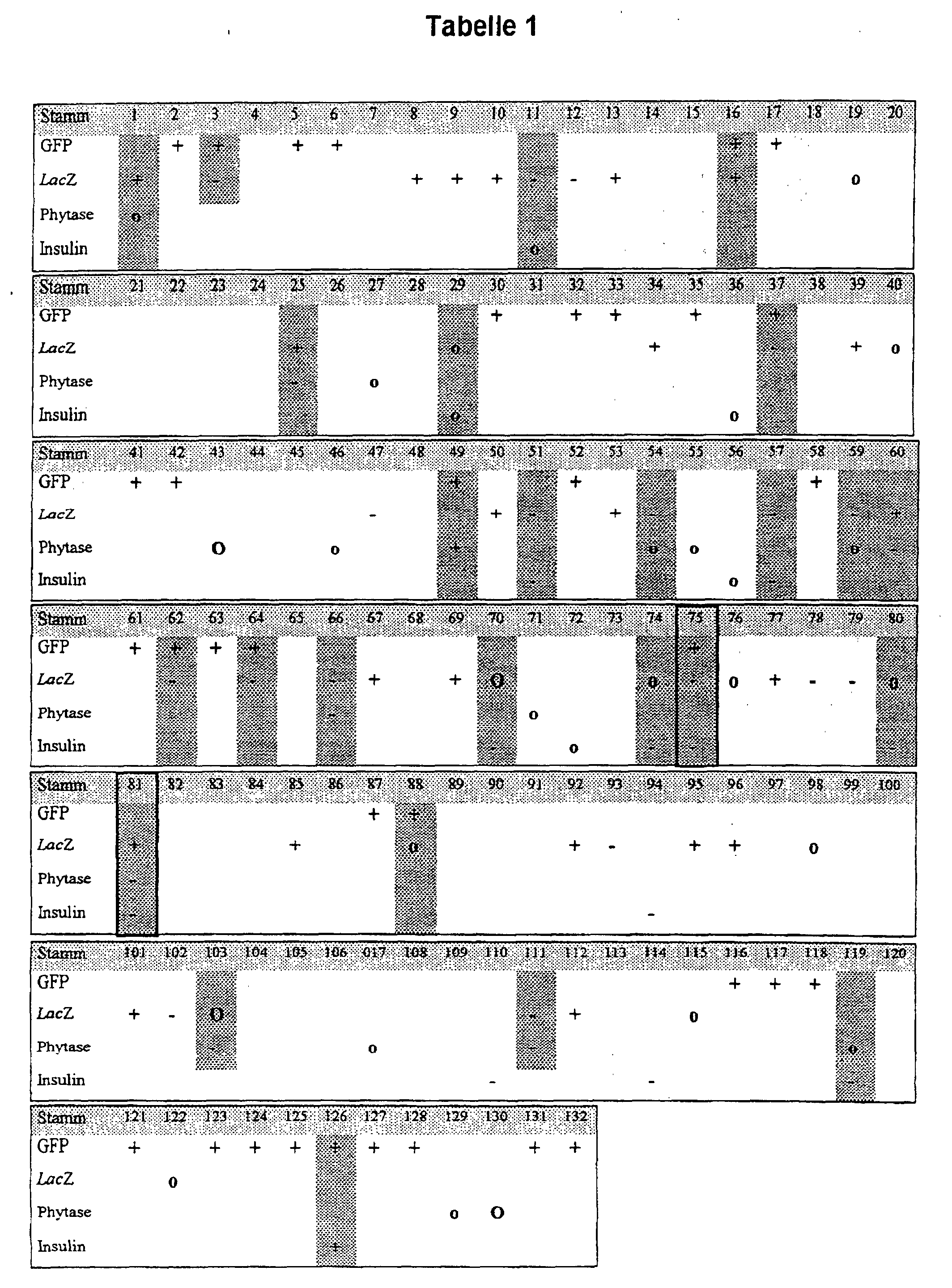

### Beispiel 17

### Stabile Phytase-Produktion

Ein rekombinanter Stamm, der durch Transformation mit dem Vektor pB14 (FMD-Promotor-kontrollierte Phytase-Produktion) erzeugt worden war, enthielt ca. 40 Kopien der rekombinanten DNA. Die mitotische Stabilität wurde über 100 Generationen Wachstum überprüft. Kulturaliquots zu Beginn der Stabilitätsuntersuchungen und nach 100 Generationen dienten zum Beimpfen von 3 ml Medium, das 2% Glyzerin als Kohlkenstoffquelle enthielt. Die sezemierte Phytase wurde nach 30stündiger Kultivierung quantifiziert. Die Konzentration wurde in beiden Fällen mit ca. 60 mg/l bestimmt.

### Literatur

Agaphonov MO, Beburov MY, Ter-Avanesyan MD, Smimov VN (1995) A disruptiondisplacement approach for the targeted integration of foreign genes in *Hansenula polymorpha*. Yeast 11:1241-1247

Bergkamp RJM, Kool IM, Geerse RH and Planta RJ (1992) Multi-copy-integration of the β-galactosidase gene from *Cyamopsis tetragonoloba* into the ribosomal DNA of *Kluyveromyces lactis*. Curr Genet 21:365-370

Casadaban MJ, Martinez-Arias A, Shapira SK. and Chon,J. (1983) β-galactosidase genefusions for analysing gene expression in *E. coli and* yeast. Meth Enzymol

Cormack, BP, Bertram G, Egerton M, Gow NAR, Falkow S, Brown AJ (1997) Yeast enhanced green fluorescent proteine (yEGFP): a reporter of gene expression in *Candida albicans*. Microbiology. 143:303-311

Gatzke R, Weydemann U, Janowicz ZA and Hollenberg CP (1995) Stable multi copy integration of vector sequences in *Hansenula polymorpha*. Appl Microbiol Biotechnol 43:377-385

Gellissen G, Piontek M, Dahlems U, Jenzelewski V, Gavagan J, DiCosimo R, Anton DA and Janowicz ZA (1996) Recombinant *Hansenula polymorpha* as a biocatalyst - coexpression of the spinach glycolate oxidase (GO) and the *S. cerevisiae* catalase T (*CTT1*) gene. Appl Microbiol Biotechnol 46:46-54

Janowicz ZA, Melber K, Merckelbach A, Jakobs E, Harford N, Comberbach M, Hollenberg CP (1991) Simultaneous expression of the S and L surface antigens of hepatitis B, and formation of mixed particles in the methylotrophic yeast *H. polymorpha*. Yeast 7:431-443

Lopes TS, Klootwijk J, Veenstra AE, van der Aar PC, van Heerikhuizen H, Raué HA and Planta RJ (1989) High-copy-number integration into the ribosomal DNA of *Saccharomyces cerevisiae*: a new vector for high-level expresssion. Gene 89:199-202

Lopes TS, Hakkaart G-JAJ, Koerts BL, Raué HA and Planta RJ (1991) Mechanism of high-copy-number integration of pMIRY-type vectors into the ribosomal DNA of *Saccharomyces cerevisiae*. Gene 105:83-90

Maniatis T, Fritsch EG, Sambrook J. (1982) Molecular Cloning: A Laboratory Manual. Cold Spring Harbor Laboratory Press, Cold Spring Harbor, New York.

Morise H,Shimomura O, Johnson FH, Winant J (1974) Intermolecular energy transfer in the bioluminecent system of *Aoquorea.* Biochemistry 4:2656-2662

Sohn JH, Choi ES, Kang HA, Rhee JS, Rhee SK (1999) A family of telomere-associated autonomously replicating sequences and their functions in targeted integration in *H. polymorpha* DL1. J Bacteriology 178:4420-4428

Weydemann U, Keup P, Piontek M, Strasser AWM, Schweden J, Gellissen G, Janowicz ZA (1995) High-level secretion of hirudin by *Hansenula polymorpha* - authentic processing of three different preprohirudins. Appl Microbiol Biotechnol 44:844-849

## Patentansprüche

1. Integrationsvektor für die Expression von mindestens einem Protein in einem Wirtsorganismus, insbesondere in einem Pilz, welcher
a) mindestens eine rDNA-Sequenz aus Hefe,
b) mindestens ein nicht-defizientes Selektionsmarkergen und
c) mindestens eine Expressionskassette
umfaßt, wobei die Expressionskassette ein im Wirtsorganismus funktionsfähiges Promotorelement, einen für ein gewünschtes Protein kodierenden Bereich eines heterologen oder genuinen Genes und ein im Wirtsorganismus funktionsfähiges Terminatorelement umfasst, **dadurch gekennzeichnet, dass** mindestens eine rDNA-Sequenz aus einem Bereich der rDNA einer methylotrophen Hefe stammt, der ein 400 bp langes Fragment aus dem 3'-Bereich des 25S-rDNA-Gens und ein 600 bp langes Fragment aus dem 5'-Bereich des 18S-rDNA-Gens sowie die dazwischen liegenden Sequenzen einschließlich der 5S-rDNA-Sequenz umfasst.

2. Integrationsvektor nach Anspruch 1, **dadurch gekennzeichnet, dass** der Wirtsorganismus ein filamentöser Pilz ist.

3. Integrationsvektor nach Anspruch 2, **dadurch gekennzeichnet, dass** der filamentöse Pilz der Gattung *Aspergillus, Neurospora, Mucor, Trichoderma, Acremonium, Sordaria* oder *Penicillium* angehört.

4. Integrationsvektor nach Anspruch 1, **dadurch gekennzeichnet, dass** der Wirtsorganismus eine Hefe ist.

5. Integrationsvektor nach Anspruch 4, **dadurch gekennzeichnet, dass** die Hefe eine methylotrophe Hefe ist.

6. Integrationsvektor nach Anspruch 4 oder 5, **dadurch gekennzeichnet, dass** die Hefe der Gattung *Saccharomyces, Hansenula, Pichia, Kluyveromyces, Schwanniomyces, Yarrowia, Arxula, Trichosporon* oder *Candida* angehört.

7. Integrationsvektor nach Anspruch 6, **dadurch gekennzeichnet, dass** die methylotrophe Hefe *Hansenula polymorpha* ist.

8. Integrationsvektor nach einem der Ansprüche 1 bis 7, **dadurch gekennzeichnet, dass** die rDNA-Sequenz ein rDNA-Fragment ist das mittels PCR mit genomischer DNA einer Hefe als Matrize und den Primern A 5'-ACG GGA TCC GGG TTT AGA CCG TCG TGA GAC AGG-3' (SEQ ID NO. 2) und B 5'-CCT GGA TCC TAC CAT CGA AAG TTG ATA GGG-3' (SEQ ID NO. 3) amplifiziert und durch Verdau mit dem Restriktionsenzym *Bam*HI erhältlich ist.

9. Integrationsvektor nach einem der Ansprüche 1 bis 8, **dadurch gekennzeichnet, dass** die rDNA-Sequenz aus einer Hefe der Gattung *Hansenula, Pichia, Saccharomyces, Kluyveromyces, Candida, Schwanniomyces, Yarrowia, Arxula* oder *Trichosporon* stammt.

10. Integrationsvektor nach Anspruch 9, **dadurch gekennzeichnet, dass** die rDNA-Sequenz aus *Hansenula polymorpha* stammt.

11. Integrationsvektor nach Anspruch 10, **dadurch gekennzeichnet, dass** die rDNA-Sequenz aus *Pichia pastoris, Pichia methanolica* oder *Candida boidinii* stammt.

12. Integrationsvektor nach einem der Ansprüche 1 bis 6, **dadurch gekennzeichnet, dass** die rDNA-Sequenz aus folgenden Sequenzen ausgewählt wird:
a) einer Sequenz nach SEQ ID NO 1;
b) einem Fragment der Sequenz nach a), das für die stabile Integration von Sequenzen des Vektors in hoher Kopienzahl in das Genom eines Wirtsorganismus, insbesondere einer Hefe geeignet ist;
c) einer Sequenz, die mit einer Sequenz nach a) oder b) zu mindestens 70% identisch ist;
d) einer Sequenz, die in dem kodierenden Bereich des 5S-Gens (Positionen 956-1074 in SEQ ID NO 1) und/oder in dem in SEQ ID NO 1 enthaltenen Abschnitt des kodierenden Bereichs des 255-Gens (Positionen 1977-2420 in SEQ ID NO 1) mit einer Sequenz nach a) oder b) zu mindestens 70% identisch ist;
e) einer Sequenz, die mit dem Gegenstrang einer Sequenz nach a) oder b) hybridisiert;
f) einem durch Substitution, Addition, Inversion und/oder Deletion einer oder mehreren Basen erhaltenen Derivat einer Sequenz nach einer der Gruppen a) bis d), das für die stabile Integration von Sequenzen des Vektors in hoher Kopienzahl in das Genom eines Wirtsorganismus, insbesondere einer Hefe geeignet ist, und
g) einer zu einer Sequenz nach einer der Gruppen a) bis f) komplementären Sequenz.

13. Integrationsvektor nach Anspruch 12, **dadurch gekennzeichnet, dass** die Sequenz nach Gruppe d) mit einer Sequenz nach der Gruppe a) oder b) zu mindestens 80% identisch ist.

14. Integrationsvektor nach Anspruch 13, **dadurch gekennzeichnet, dass** die Sequenz nach der Gruppe d) mit einer Sequenz nach der Gruppe a) oder b) zu mindestens 90% identisch ist.

15. Integrationsvektor nach einem der Ansprüche 1 bis 14, **dadurch gekennzeichnet, dass** er ein zusätzliches Selektionsmarkergen für *E. coli* umfaßt.

16. Integrationsvektor nach einem der Ansprüche 1 bis 15 mit einer Länge zwischen 7 und 12 Kb.

17. Integrationsvektor nach einem der Ansprüche 1 bis 16, **dadurch gekennzeichnet, dass** er Sequenzen enthält, die ein oder mehrere Polypeptide kodieren, die eine verbesserte Sezemierung eines kointegrierten rekombinanten sekretorischen Proteins aus einem Wirtsorganismus ermöglichen.

18. Integrationsvektor nach Anspruch 17, **dadurch gekennzeichnet, dass** die Sequenzen ein Polypeptid mit der biologischen Aktivität einer Ca²⁺/Calmodulin-abhängigen Proteinkinase und/oder ein Polypeptid mit der biologischen Aktivität eines Translationsinitiationsfaktors 4E (eIF4E) kodieren.

19. Integrationsvektor nach Anspruch 18, **dadurch gekennzeichnet, dass** die Sequenzen den kodierenden Bereich des *CMK2*-Gens und/oder den kodierenden Bereich des *CDC33*-Gens aus *Saccharomyces cerevisiae* umfassen.

20. Wirtsorganismus, der mindestens einen Integrationsvektor nach einem der Ansprüche 1 bis 19 stabil im Genom integriert umfaßt.

21. Wirtsorganismus nach Anspruch 20, **dadurch gekennzeichnet, dass** er ein filamentöser Pilz ist.

22. Wirtsorganismus nach Anspruch 21, **dadurch gekennzeichnet, dass** er der Gattung *Aspergillus, Neurospora, Mucor, Trichoderma, Acremonium, Sordaria* oder *Penicillium* angehört.

23. Wirtsorganismus nach Anspruch 22, **dadurch gekennzeichnet dass** er eine Hefe ist

24. Wirtsorganismus nach Anspruch 23, **dadurch gekennzeichnet, dass** die Hefe eine methylotrophe Hefe ist.

25. Wirtsorganismus nach Anspruch 23 oder 24, **dadurch gekennzeichnet, dass** er der Gattung *Saccharomyces, Hansenula, Pichia, Kluyveromyces, Schwanniomyces, Yarrowia, Arxula, Trichosporon* oder *Candida* angehört.

26. Wirtsorganismus nach Anspruch 25, **dadurch gekennzeichnet, dass** die Hefe *Hansenula polymorpha* ist.

27. Wirtsorganismus, der durch stabile Integration von einem oder mehreren Integrationsvektoren nach einem der Ansprüche 1 bis 15 in das Genom eines Pilzes erhältlich ist.

28. Wirtsorganismus nach Anspruch 27, **dadurch gekennzeichnet, dass** der Pilz ein filamentöser Pilz ist.

29. Wirtsorganismus nach Anspruch 28, **dadurch gekennzeichnet, dass** der filamentöse Pilz der Gattung *Aspergillus, Neurospora, Mucor, Trichoderma, Acremonium, Sorda* *ria* oder *Penicillium* angehört.

30. Wirtsorganismus nach Anspruch 29, **dadurch gekennzeichnet, dass** der Pilz eine Hefe ist.

31. Wirtsorganismus nach Anspruch 30, **dadurch gekennzeichnet, dass** die Hefe eine methylotrophe Hefe ist.

32. Wirtsorganismus nach Anspruch 30 oder 31, **dadurch gekennzeichnet, dass** die Hefe der Gattung *Saccharomyces, Hansenula, Pichia, Kluyveromyces, Schwanniomyces, Yarrowia, Arxula, Trichosporon* oder *Candida* angehört.

33. Wirtsorganismus nach Anspruch 32, **dadurch gekennzeichnet, dass** die Hefe *Hansenula polymorpha* ist.

34. Wirtsorganismus nach einem der Ansprüche 27 bis 31, **dadurch gekennzeichnet, dass** er durch Integration mehrerer Vektoren, die voneinander unterschiedliche Expressionkassetten umfassen, erhältlich ist.

35. Verfahren zur Herstellung von einem oder mehreren Proteinen, **dadurch gekennzeichnet, dass** ein Wirtsorganismus nach einem der Ansprüche 20 bis 34 in an sich bekannter Weise kultiviert und das erzeugte Protein gewonnen wird.

36. Verwendung eines Integrationsvektors nach einem der Ansprüche 1 bis 20 zur Herstellung von einem oder mehreren Proteinen in einem Wirtsorganismus nach einem der Ansprüche 20 bis 34.

37. Verwendung eines Wirtsorganismus nach einem der Ansprüche 20 bis 34 zur Herstellung von einem oder mehreren Proteinen.

## Claims

1. Integration vector for expressing at least one protein in a host organism, in particular in a, fungus, which vector comprises
a) at least one yeast rDNA sequence,
b) at least one non-deficient selection marker gene and
c) at least one expression cassette,
the expression cassette comprising a promoter element which is functional in the host organism, a region of a heterologous or genuine gene, which codes for a desired protein, and a terminator element which is functional in the host organism, **characterized in that** at least one rDNA sequence is derived from a region of the rDNA of a methylotrophic yeast, which region comprises a 400 bp fragment from the 3' region of the 25S rDNA gene and a 600 bp fragment from the 5' region of the 18S rDNA gene and the sequences located in between, including the 5S rDNA sequence.

2. Integration vector according to Claim 1, **characterized in that** the host organism is a filamentous fungus.

3. Integration vector according to Claim 2, **characterized in that** the filamentous fungus belongs to the genus *Aspergillus, Neurospora, Mucor, Trichoderma, Acremonium, Sordaria* or *Penicillium.*

4. Integration vector according to Claim 1, **characterized in that** the host organism is a yeast.

5. Integration vector according to Claim 4, **characterized in that** the yeast is a methylotrophic yeast.

6. Integration vector according to Claim 4 or 5, **characterized in that** the yeast belongs to the genus *Saccharomyces, Hansenula, Pichia, Kluyveromyces, Schwanniomyces, Yarrowia, Arxula, Trichosporon* or *Candida*.

7. Integration vector according to Claim 6, **characterized in that** the methylotrophic yeast is *Hansenula polymorpha*.

8. Integration vector according to any of Claims 1 to 7, **characterized in that** the rDNA sequence is an rDNA fragment which is amplified by means of PCR with a yeast genomic DNA as template and the primers A 
5′-ACG GGA TCC GGG TTT AGA CCG TCG TGA GAC AGG-3′
(SEQ ID NO. 2)
 and B 
5'-CCT GGA TCC TAC CAT CGA AAG TTG ATA GGG-3' (SEQ
ID NO. 3)
 and can be obtained by digestion with the restriction enzyme *Bam*HI.

9. Integration vector according to any of Claims 1 to 8, **characterized in that** the rDNA sequence is derived from a yeast of the genus *Hansenula, Pichia, Saccharomyces, Kluyveromyces, Candida, Schwanniomyces, Yarrowia, Arxula* or *Trichosporon*.

10. Integration vector according to Claim 9, **characterized in that** the rDNA sequence is derived from *Hansenula polymorpha*.

11. Integration vector according to Claim 10, **characterized in that** the rDNA sequence is derived from *Pichia pastoris, Pichia methanolica* or *Candida boidinii*.

12. Integration vector according to any of Claims 1 to 6, **characterized in that** the rDNA sequence is selected from the following sequences:
a) a sequence according to SEQ ID NO. 1;
b) a fragment of the sequence of a), which is suitable for the stable integration of a large number of copies of sequences of the vector into the genome of a host organism, in particular of a yeast;
c) a sequence which is at least 70% identical to a sequence of a) or b);
d) a sequence which is at least 70% identical to a sequence of a) or b) in the coding region of the 5S gene (positions 956-1074 in SEQ ID NO. 1) and/or in the section of the coding region of the 25S gene, contained in SEQ ID NO. 1 (positions 1977-2420 in SEQ ID NO. 1);
e) a sequence which hybridizes with the counter-strand of a sequence of a) or b);
f) a derivative of a sequence of any of groups a) to d), which derivative is obtained by substitution, addition, inversion and/or deletion of one or more bases and which is suitable for the stable integration of a large number of copies of sequences of the vector into the genome of a host organism, in particular of a yeast; and
g) a sequence which is complementary to a sequence of any of groups a) to f).

13. Integration vector according to Claim 12, **characterized in that** the sequence of group d) is at least 80% identical to a sequence of group a) or b).

14. Integration vector according to Claim 13, **characterized in that** the sequence of group d) is at least 90% identical to a sequence of group a) or b).

15. Integration vector according to any of Claims 1 to 14, **characterized in that** it comprises an additional selection marker gene for *E. coli*.

16. Integration vector according to any of Claims 1 to 15, having a length of between 7 and 12 kb.

17. Integration vector according to any of Claims 1 to 16, **characterized in that** it comprises sequences encoding one or more polypeptides which make possible improved secretion of a co-integrated recombinant secretory protein from a host organism.

18. Integration vector according to Claim 17, **characterized in that** the sequences encode a polypeptide having the biological activity of a Ca²⁺/calmodulin-dependent protein kinase and/or a polypeptide having the biological activity of a translation initiation factor 4E (eIF4E).

19. Integration vector according to Claim 18, **characterized in that** the sequences comprise the coding region of the *CMK2* gene and/or the coding region of the *CDC33* gene from *Saccharomyces cerevisiae.*

20. Host organism which comprises at least one integration vector according to any of Claims 1 to 19 stably integrated in the genome.

21. Host organism according to Claim 20, **characterized in that** it is a filamentous fungus.

22. Host organism according to Claim 21, **characterized in that** it belongs to the genus *Aspergillus, Neurospora, Mucor, Trichoderma, Acremonium, Sordaria* or *Penicillium*.

23. Host organism according to Claim 22, **characterized in that** it is a yeast.

24. Host organism according to Claim 23, **characterized in that** the yeast is a methylotrophic yeast.

25. Host organism according to Claim 23 or 24, **characterized in that** it belongs to the genus *Saccharomyces, Hansenula, Pichia, Kluyveromyces, Schwanniomyces, Yarrowia, Arxula, Trichosporon* or *Candida*.

26. Host organism according to Claim 25, **characterized in that** the yeast is *Hansenula polymorpha*.

27. Host organism which is obtainable by stable integration of one or more integration vectors according to any of Claims 1 to 15 into the genome of a fungus.

28. Host organism according to Claim 27, **characterized in that** the fungus is a filamentous fungus.

29. Host organism according to Claim 28, **characterized in that** the filamentous fungus belongs to the genus *Aspergillus, Neurospora, Mucor, Trichoderma, Acremonium, Sordaria* or *Penicillium*.

30. Host organism according to Claim 29, **characterized in that** the fungus is a yeast.

31. Host organism according to Claim 30, **characterized in that** the yeast is a methylotrophic yeast.

32. Host organism according to Claim 30 or 31, **characterized in that** the yeast belongs to the genus *Saccharomyces, Hansenula, Pichia, Kluyveromyces, Schwanniomyces, Yarrowia, Arxula, Trichosporon* or *Candida*.

33. Host organism according to Claim 32, **characterized in that** the yeast is *Hansenula polymorpha*.

34. Host organism according to any of Claims 27 to 31, **characterized in that** it is obtainable by integration of a plurality of vectors which comprise expression cassettes differing from one another.

35. Method for preparing one or more proteins, **characterized in that** a host organism according to any of Claims 20 to 34 is cultured in a manner known per se and the protein produced is isolated.

36. Use of an integration vector according to any of Claims 1 to 20 for preparing one or more proteins in a host organism according to any of Claims 20 to 34.

37. Use of a host organism according to any of Claims 20 to 34 for preparing one or more proteins.

## Revendications

1. Vecteur d'intégration pour l'expression d'au moins une protéine dans un organisme hôte, en particulier dans un champignon, qui comprend
a) au moins une séquence d'ADNr de levure,
b) au moins un marqueur génétique de sélection non déficient et
c) au moins une cassette d'expression,
dans lequel la cassette d'expression comprend un élément promoteur fonctionnel dans l'organisme hôte, un domaine d'un gène hétérologue ou d'origine codant une protéine souhaitée et un élément terminateur fonctionnel dans l'organisme hôte, **caractérisé en ce qu'**au moins une séquence d'ADNr provient d'un domaine de l'ADNr d'une levure méthylotrophe qui comprend un fragment long de 400 pb du domaine 3' du gène 25S-ADNr et un fragment long de 600 pb du domaine 5' du gène 18S-ADNr ainsi que les séquences se trouvant entre ces fragments, y compris la séquence 5S-ADNr.

2. Vecteur d'intégration selon la revendication 1, **caractérisé en ce que** l'organisme hôte est un champignon filamenteux.

3. Vecteur d'intégration selon la revendication 2, **caractérisé en ce que** le champignon filamenteux appartient à l'espèce *Aspergillus, Neurospora, Mucor, Trichoderma, Acremonium, Sordaria* ou *Penicillium*.

4. Vecteur d'intégration selon la revendication 1, **caractérisé en ce que** l'organisme hôte est une levure.

5. Vecteur d'intégration selon la revendication 4, **caractérisé en ce que** la levure est une levure méthylotrophe.

6. Vecteur d'intégration selon la revendication 4 ou la revendication 5, **caractérisé en ce que** la levure appartient à l'espèce *Saccharomyces, Hansenula, Pichia, Kluyveromyces, Schwanniomyces, Yarrowia, Arxula, Trichosporon* ou *Candida*.

7. Vecteur d'intégration selon la revendication 6, **caractérisé en ce que** la levure méthylotrophe est *Hansenula polymorpha*.

8. Vecteur d'intégration selon l'une quelconque des revendications 1 à 7, **caractérisé en ce que** la séquence d'ADNr est un fragment d'ADNr qui est amplifié par PCR avec l'ADN génomique d'une levure comme matrice et les amorces A 5'-ACG GGA TCC GGG TTT AGA CCG TCG TGA GAC AGG-3' (SEQ ID NO. 2) et B 5'-CCT GGA TCC TAC CAT CGA AAG TTG ATA GGG-3' (SEQ ID NO. 3) et qui peut être obtenu par digestion avec l'enzyme de restriction *Bam*HI.

9. Vecteur d'intégration selon l'une quelconque des revendications 1 à 8, **caractérisé en ce que** la séquence d'ADNr provient d'une levure de l'espèce *Hansenula, Pichia, Saccharomyces, Kluyveromyces, Candida, Schwanniomyces, Yarrowia, Arxula* ou *Trichosporon.*

10. Vecteur d'intégration selon la revendication 9, **caractérisé en ce que** la séquence d'ADNr provient de *Hansenula polymorpha*.

11. Vecteur d'intégration selon la revendication 10, **caractérisé en ce que** la séquence d'ADNr provient de *Pichia pastoris, Pichia methanolica* ou *Candida boidinii*.

12. Vecteur d'intégration selon l'une quelconque des revendications 1 à 6, **caractérisé en ce que** la séquence d'ADNr est choisie parmi les séquences suivantes :
a) une séquence selon SEQ ID N° 1 ;
b) un fragment de la séquence selon a) qui est adapté pour l'intégration stable de séquences du vecteur en un grand nombre de copies dans le génome d'un organisme hôte, en particulier une levure ;
c) une séquence qui est identique à une séquence selon a) ou b) à au moins 70 % ;
d) une séquence qui est identique dans le domaine codant du gène 5S (positions 956-1074 de SEQ ID N°1) et/ou dans la partie contenant SEQ ID N° 1 du domaine codant du gène 25S (positions 1977-2420 de SEQ ID N° 1) à une séquence selon a) ou b) à au moins 70 % ;
e) une séquence qui s'hybride avec le brin inverse d'une séquence selon a) ou b) ;
f) un dérivé, obtenu par substitution, addition, inversion et/ou délétion d'une ou plusieurs bases, d'une séquence selon un des groupes a) à d) qui est adapté pour l'intégration stable de séquences du vecteur en un grand nombre de copies dans le génome d'un organisme hôte, en particulier une levure, et
g) une séquence complémentaire d'une séquence selon l'un des groupes a) à f).

13. Vecteur d'intégration selon la revendication 12, **caractérisé en ce que** la séquence selon le groupe d) est identique à une séquence selon le groupe a) ou b) à au moins 80 %.

14. Vecteur d'intégration selon la revendication 13, **caractérisé en ce que** la séquence selon le groupe d) est identique à une séquence selon le groupe a) ou b) à au moins 90 %.

15. Vecteur d'intégration selon l'une quelconque des revendications 1 à 14, **caractérisé en ce qu'**il comprend un gène marqueur de sélection supplémentaire pour *E. coli*.

16. Vecteur d'intégration selon l'une quelconque des revendications 1 à 15 d'une longueur comprise entre 7 et 12 kb.

17. Vecteur d'intégration selon l'une quelconque des revendications 1 à 16, **caractérisé en ce qu'**il contient des séquences qui codent un ou plusieurs polypeptides qui permettent une meilleure sécrétion d'une protéine sécrétrice recombinante co-intégrée par un organisme hôte.

18. Vecteur d'intégration selon la revendication 17, **caractérisé en ce que** les séquences codent un polypeptide doté de l'activité biologique d'une protéine kinase Ca²⁺/calmoduline dépendante et/ou un polypeptide doté de l'activité biologique d'un facteur d'initiation de la traduction 4E (eIF4E).

19. Vecteur d'intégration selon la revendication 18, **caractérisé en ce que** les séquences comprennent le domaine codant du gène *CMK2* et/ou le domaine codant du gène *CDC33* de *Saccharomyces cerevisiae.*

20. Organisme hôte qui comprend au moins un vecteur d'intégration selon l'une quelconque des revendications 1 à 19 intégré de manière stable dans son génome.

21. Organisme hôte selon la revendication 20, **caractérisé en ce qu'**il s'agit d'un champignon filamenteux.

22. Organisme hôte selon la revendication 21, **caractérisé en ce qu'**il appartient à l'espèce *Aspergillus, Neurospora, Mucor, Trichoderma, Acremonium, Sordaria* ou *Penicillium.*

23. Organisme hôte selon la revendication 22, **caractérisé en ce qu'**il s'agit d'une levure.

24. Organisme hôte selon la revendication 23, **caractérisé en ce que** la levure est une levure méthylotrophe.

25. Organisme hôte selon la revendication 23 ou 24, **caractérisé en ce qu'**il appartient à l'espèce *Saccharomyces, Hansenula, Pichia, Kluyveromyces*, *Schwanniomyces, Yarrowia, Arxula, Trichosporon* ou *Candida*.

26. Organisme hôte selon la revendication 25, **caractérisé en ce que** la levure est *Hansenula polymorpha*.

27. Organisme hôte qui peut être obtenu par intégration stable d'un ou plusieurs vecteurs d'intégration selon l'une quelconque des revendications 1 à 15 dans le génome d'un champignon.

28. Organisme hôte selon la revendication 27, **caractérisé en ce que** le champignon est un champignon filamenteux.

29. Organisme hôte selon la revendication 28, **caractérisé en ce que** le champignon filamenteux appartient à l'espèce *Aspergillus, Neurospora, Mucor, Trichoderma, Acremonium, Sordaria* ou *Penicillium*.

30. Organisme hôte selon la revendication 29, **caractérisé en ce que** le champignon est une levure.

31. Organisme hôte selon la revendication 30, **caractérisé en ce que** la levure est une levure méthylotrophe.

32. Organisme hôte selon la revendication 30 ou la revendication 31, **caractérisé en ce que** la levure appartient à l'espèce *Saccharomyces, Hansenula, Pichia, Kluyveromyces, Schwanniomyces, Yarrowia, Arxula, Trichosporon* ou *Candida*.

33. Organisme hôte selon la revendication 32, **caractérisé en ce que** la levure est *Hansenula polymorpha*.

34. Organisme hôte selon l'une quelconque des revendications 27 à 31, **caractérisé en ce qu'**il peut être obtenu par intégration de plusieurs vecteurs comprenant des cassettes d'expression différentes les unes des autres.

35. Procédé pour la fabrication d'une ou plusieurs protéines, **caractérisé en ce qu'**un organisme hôte selon l'une quelconque des revendications 20 à 34 est cultivé de manière connue en soi et **en ce que** la protéine produite est récoltée.

36. Utilisation d'un vecteur d'intégration selon l'une quelconque des revendications 1 à 20 pour la fabrication d'une ou plusieurs protéines dans un organisme hôte selon l'une quelconque des revendications 20 à 34.

37. Utilisation d'un organisme hôte selon l'une quelconque des revendications 20 à 34 pour la fabrication d'une ou plusieurs protéines.
